(19) Europäisches Patentamt
European Patent Office
Office européen des brevets

(11) EP 3 393 595 B1

(12) **EUROPÄISCHE PATENTSCHRIFT**

(45) Veröffentlichungstag und Bekanntmachung des Hinweises auf die Patenterteilung:
**23.06.2021 Patentblatt 2021/25**

(21) Anmeldenummer: **16805388.2**

(22) Anmeldetag: **01.12.2016**

(51) Int Cl.:
***A61Q 5/08*** *(2006.01)* ***A61Q 5/10*** *(2006.01)*
***A61K 8/19*** *(2006.01)* ***A61K 8/26*** *(2006.01)*
***A61K 8/73*** *(2006.01)*

(86) Internationale Anmeldenummer:
**PCT/EP2016/079382**

(87) Internationale Veröffentlichungsnummer:
**WO 2017/108364 (29.06.2017 Gazette 2017/26)**

(54) **VERFAHREN ZUR MULTITONALEN FARBVERÄNDERUNG KERATINISCHER FASERN**

METHOD FOR MULTITONAL COLORCHANGE OF KERATINIC FIBERS

METHODE POUR LA COLORATION MULTITON DES FIBRES KERATINIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2015 DE 102015226171**

(43) Veröffentlichungstag der Anmeldung:
**31.10.2018 Patentblatt 2018/44**

(73) Patentinhaber: **Henkel AG & Co. KGaA**
**40589 Düsseldorf (DE)**

(72) Erfinder:
- **MANNECK, Hartmut**
  **23858 Barnitz (DE)**
- **HOEPFNER, Stefan**
  **22523 Hamburg (DE)**
- **SCHWEINSBERG, Matthias**
  **40764 Langenfeld (DE)**

(56) Entgegenhaltungen:
**EP-A2- 2 574 331** **WO-A1-2014/164213**
**WO-A1-2014/187575** **WO-A1-2015/028015**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 393 595 B1

## Beschreibung

Technischer Hintergrund

Für permanente, intensive Färbungen keratinischer Fasern, insbesondere von Humanhaar, mit entsprechenden Echtheitseigenschaften werden so genannte Oxidationsfärbemittel verwendet. Solche Färbemittel enthalten üblicherweise Oxidationsfarbstoffvorprodukte, so genannte Entwicklerkomponenten und Kupplerkomponenten, die unter dem Einfluss von Oxidationsmitteln oder von Luftsauerstoff miteinander reagieren, insbesondere oligomerisieren, und dadurch die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, die als färbende Komponente so genannte direktziehende Farbstoffe ("Direktzieher") enthalten.

Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Sollen Substrate aufgehellt oder gar gebleicht werden, werden die das Substrat färbenden Farbstoffe, beispielsweise das Haar-eigene Melanin, meist oxidativ unter Einsatz von entsprechenden Oxidationsmitteln, wie Wasserstoffperoxid, entfärbt.

Bei der Haarfärbung - insbesondere bei der Haarfärbung in Heimanwendung durch den Verbraucher selbst - tritt das Problem auf, dass natürliche Farbnuancierungen vollständig überdeckt werden, so dass multitonale Färbungen schwer zu realisieren sind.

Um dem Haar ein natürlicheres Aussehen zu verleihen, ist es im Stand der Technik bekannt, ungefärbte wie auch bereits gefärbte Haare durch die gezielte Anwendung von Oxidationsmitteln partiell zu entfärben. Die Haarpartien ("Strähnchen"), auf die die Oxidationsmittel aufgetragen werden, bleichen dabei mindestens anteilsweise aus, woraus eine multitonale Haarfarbe resultiert. Auch die Färbung einzelner Haarpartien ("Strähnchen") mit einer anderen Farbe ist im Stand der Technik bekannt. Die Applikation des ggf. farbstoffhaltigen Oxidationsmittels erfolgt dabei mit einer Bürste oder einem Pinsel, wobei die nicht zu behandelnden Haare mit festen flächigen Trennhilfen, insbesondere mit Folien aus festen Materialien, wie Aluminium, Papier oder Styropor und ähnlichen Materialien, oder mit einer so genannten "Strähnchenhaube" vor dem Kontakt mit dem ggf. farbstoffhaltigen Oxidationsmittel geschützt werden. Eine derartige Strähnchenhaube ist beispielsweise in WO 2007/087978 offenbart.

Diese Applikationsart löst zwar das Problem der möglichst natürlichen Färbung von Haaren, lässt aber nur das Setzen von "Highlights" zu. Für "Lowlights", d. h. dunklere Partien, müsste nachgefärbt werden. In den zuvor beschriebenen Fällen wäre daher ein zeitaufwendiger zweiter Entfärbe- bzw. Färbeschritt erforderlich, der sich an die erste Färbung anschließt. Insbesondere bei der Heimanwendung müsste daher zunächst das gesamte Haar coloriert werden, bevor die Anwenderin "Highlights" oder "Lowlights" setzen kann. Von vielen Verbraucherinnen wird dies als zu zeitaufwändig und auch als frustrierend empfunden, da der wesentliche farbverändernde Schritt anfangs erfolgt und in einem zweiten Schritt lediglich "korrigiert" wird.

Bei den Strähnchenfärbeverfahren des Standes der Technik fallen die Folien oder die Strähnchenhaube am Ende des Verfahrens als fester Abfall an, der entsorgt werden muss. Dies ist mit einer entsprechenden Umweltbelastung verbunden. Der Umgang mit den Folien ist teilweise schwierig; z. B. wird der Zugang zu noch nicht behandelten Haaren durch bereits liegende Folien behindert. Darüber hinaus besteht insbesondere bei Folien aus Papier, Plastik und Styropor, die Gefahr, dass sie auf dem behandelten Haar schlecht haften und abrutschen.

Bei einigen Folientypen und Anwendungen kann es zur Überhitzung kommen, weil die Möglichkeit, die Reaktionswärme des exothermen Oxidationsprozesses durch Verdunstung flüchtiger Rezepturbestandteile, wie beispielsweise Wasser, abzuführen, durch die dampfundurchlässigen Folien stark eingeschränkt ist.

Die Produktion von kommerziellen Haarfärbemitteln steht, wie die Produktion anderer Masseprodukte auch, unter hohem Kostendruck. Der Fachmann ist daher bemüht, möglichst viele Rezepturbestandteile zu vereinheitlichen. Bei Färbecremes wird beispielsweise gern mit einer bestimmten Emulsionsbasis als "Träger" gearbeitet. Diejenigen Zusatzstoffe, die einen individualisierten Produktnutzen liefern - seien es die Farbpulvermischungen für eine bestimmte Nuance oder ausgewählte Pflegestoffe, um unterschiedliche Haarqualitäten in optimaler Weise zu behandeln -, müssen mit dem Träger, z. B. der Färbecremebasis, so kompatibel wie möglich sein. Als Kriterium für Kompatibilität dient dabei die so genannte Farbverschiebung. Die Zugabe individualisierter Zusatzstoffe kann dazu führen, dass Farbunterschiede zwischen der mit dem Zusatzstoff-freien (Standard)Träger und der mit dem Zusatzstoff-haltigen Träger erzielten Haarfärbung auftreten. Derartige Farbunterschiede werden im Sinne der vorliegenden Anmeldung als "Farbverschiebung" bezeichnet. Diese Farbverschiebung, auch als dE oder ΔE bezeichnet, lässt sich farbmetrisch gut mit einem Farbmessgerät, bestimmen, mit dem die Farben im L*,a*,b*-Farbraum vermessen werden, beispielsweise mit einem Farbmessgerät der Firma Datacolor, Typ Spectraflash 450.

Unter dem L*,a*,b*-Farbraum wird der CIELAB-Farbenraum verstanden. Der L-Wert steht dabei für die Helligkeit der Färbung (schwarz-weiß-Achse); je größer der Wert für L ist, desto heller ist die Färbung. Der a-Wert steht für die rot-grün-Achse des Systems; je größer dieser Wert ist, desto mehr ist die Färbung ins Rote verschoben. Der b-Wert

steht für die gelb-blau-Achse des Systems; je größer dieser Wert ist, umso mehr ist die Färbung ins Gelbe verschoben.

Die Farbverschiebung ΔE, also die Farbdifferenz zwischen zwei (Haar-)Farben, für die jeweils eine L*,a*,b*-Wertekombination bestimmt wurde, wird gemäß folgender Formel berechnet:

$$\Delta E = (\Delta L^2 + \Delta a^2 + \Delta b^2)^{0,5}$$

Je größer der Wert für ΔE ist, desto stärker ausgeprägt ist die Farbdifferenz oder "Farbverschiebung". Farbdifferenzen mit ΔE < 1 sind für das menschliche Auge nicht wahrnehmbar. Farbdifferenzen mit ΔE < 2 sind für das geschulte Auge sichtbar. Farbdifferenzen mit ΔE > 2 sind auch für das ungeschulte Auge sichtbar.

Im ungünstigsten Fall bewirkt die Zugabe eines Zusatzstoffs zu einem Färbemittelträger eine Farbverschiebung gegenüber dem Zusatzstoff-freien Träger (Standard) mit ΔE > 2, die auch für das ungeschulte Auge des Verbrauchers sichtbar ist. Um zu vermeiden, dass bei jeder Zusatzstoff-Änderung des Standardträgers aufwändige Tests hinsichtlich der erzielbaren Haarfärbung und ggf. der Echtheitseigenschaften durchgeführt werden müssen, ist es daher wünschenswert, Wirk- und Pflegestoffe für das Haar zu identifizieren, deren Zugabe keine oder zumindest nur eine geringe Farbverschiebung bewirkt.

Die Offenlegungsschriften EP2574331A2 und WO2014164213A1 offenbaren Färbeverfahren, die multitonale Färbungen in einem Färbeschritt ermöglichen, bei dem einem Haarfärbemittel vor der Applikation auf ausgewählte Haarpartien oder Strähnen ein Verdickungsmittel, insbesondere ein anionisches oder kationisches Assoziativpolymer (EP2574331A2), das verdickend wirkt, in höherer Konzentration zugesetzt wird. Das Färbemittel verdickt auf den Haarsträhnen zu einer hoch viskosen Paste, wodurch eine Trennwirkung zu benachbarten Haarpartien oder Strähnen erzielt wird. Der Farbaustausch zwischen benachbarten Strähnen, die mit unterschiedlichen Färbe- oder Bleichmitteln behandelt wurden, wird so minimiert. Über die gesamte Einwirkzeit der Färbemittel von ca. 30 bis 60 Minuten hinweg lässt sich die Farbstoffdiffusion zwischen benachbarten Strähnen, die in physischem Kontakt zueinander stehen, allerdings nicht vollständig vermeiden. Das entsprechende Handelsprodukt für die professionelle Salonanwendung wird daher mit dem Hinweis vermarktet, dass der Farbunterschied zwischen benachbarten Strähnen nicht mehr als 3 Farbtonstufen betragen sollte. Andernfalls würde das Färbeergebnis durch einen sichtbaren Farbaustausch beeinträchtigt.

## Aufgabe

Der vorliegenden Erfindung lag die Aufgabe zugrunde, ein Verfahren bereitzustellen, das multitonale Färbungen in einem Färbeschritt ermöglicht. Dabei sollte die Färbung des Haares mit der Erzeugung von "Highlights" oder "Lowlights" einhergehen, damit nach dem Ausspülen des Färbemittels direkt ein Ergebnis sichtbar ist. Eine weitere Aufgabe der vorliegenden Erfindung war es, ein möglichst ressourcenschonendes multitonales Färbeverfahren bereitzustellen, das ohne festen Folienabfall und möglichst nur mit Materialien aus nachwachsenden Rohstoffen auf nicht-fossiler Rohstoffbasis auskommt. Bei dieser Aufgabenstellung war es besonders wichtig, dass die Folienersatzstoffe ebenso wie die flächigen Folien eine hohe Trennkraft aufweisen, das heißt, eine möglichst saubere Trennung der unterschiedlich gefärbten oder gebleichten Faserpartien oder Fasersträhnen erlauben, ohne dass es zu einem Farbaustausch zwischen benachbarten Strähnen kommt.

Der vorliegenden Anmeldung lag weiterhin die Aufgabe zu Grunde, ein Verfahren zur oxidativen Haarfärbung bereitzustellen, das multitonale Färbungen in einem Färbeschritt ermöglicht, wobei die Zusatzstoffe, die die Folien ersetzen, keine oder nur eine minimale Farbverschiebung hervorrufen. Eine weitere Aufgabe der vorliegenden Erfindung war es, ein multitonales Blondier- oder Aufhellverfahren bereitzustellen, das eine vereinfachte optische Kontrolle der Entfärbungsentwicklung oder Farbentwicklung ermöglicht.

Gegenstand der vorliegenden Anmeldung ist ein Verfahren zur Behandlung keratinischer Fasern, das es erlaubt, in einer einzigen Behandlungssitzung das Haar zu colorieren und gleichzeitig eine multitonale Färbung mit verschiedenfarbigen Partien (Strähnchen), insbesondere helleren ("highlights") oder dunkleren ("lowlights") Partien (Strähnchen), zu erzeugen, wobei auf den Einsatz von festen flächigen Trennhilfen, insbesondere von Folien aus festen Materialien, wie Aluminium, Papier oder Styropor und ähnlichen Materialien verzichtet werden kann.

Hierzu wird dem anwendungsbereiten Färbe- oder Aufhellmittel, das üblicherweise einen stärker alkalischen pH-Wert aufweist, oder seinen einzelnen Komponenten zunächst ein ausgewähltes Polysaccharid zugemischt, dessen Verdickungsleistung in geringerem Maße pH-Wert-abhängig ist als die Verdickungsleistung von beispielsweise (Meth)Acrylsäure-, (Meth)Acrylsäureester-, (Meth)Acrylamid- und/oder 2-Acrylamido-2-methylpropansulfonsäure-Polymeren. Anstatt dieses ausgewählte Polysaccharid dem anwendungsbereiten Färbe- oder Aufhellmittel oder seinen einzelnen Komponenten zuzumischen, kann zunächst auch das anwendungsbereite Färbe- oder Aufhellmittel auf ausgewählte Keratinfaserpartien, insbesondere Haarpartien, oder Keratinfasersträhnen, insbesondere Haarsträhnen, in üblicher Weise aufgetragen und anschließend mit einer wässrigen Lösung des ausgewählten Polysaccharids besprüht oder bestrichen werden. Anschließend werden die mit Polysaccharid behandelten Faserpartien oder Strähnen mit einer

wässrigen Lösung eines ausgewählten Salzes besprüht oder bestrichen, dessen Kationen das ausgewählte Polysaccharid zu einem filmförmigen Gel verfestigen. Dieser Film wirkt, ähnlich wie die Strähnchenfolien des bekannten Verfahrens, als Trennschicht, die es erlaubt, benachbarte Faserpartien oder Strähnen verschiedenfarbig zu färben oder zu bleichen. Darüber hinaus wirkt dieser Film als durchsichtige Barriere, die eine einfache optische Kontrolle der Entwicklung des Blondier- oder Aufhelleffekts während der Einwirkzeit ermöglicht.

Prinzipiell ist das erfindungsgemäße Verfahren auch zur monotonalen Farbveränderung von Keratinfasern geeignet. So kann man sich die Polysaccharid-Filmbildung auch zu Nutze machen, um das unerwünschte Eintrocknen des Aufhell- oder Färbemittels oder die Freisetzung von Ammoniak während der Einwirkzeit auf den Keratinfasern zu verlangsamen. Dabei kann das gesamte Keratinfaserensemble, z. B. das gesamte Haupthaar, einheitlich ohne partielle, z.B. strähnenförmige, Variation der Farbtöne mit der Färbe- oder Aufhellzusammensetzung beaufschlagt und anschließend der Filmbildungsprozedur unterzogen werden.

Unter den Begriffen "keratinische Fasern" und "Keratinfasern" sind erfindungsgemäß bevorzugt menschliche Haare, daneben aber auch Pelze, Wolle und Federn zu verstehen.

Unter "anschließend" wird erfindungsgemäß bevorzugt ein Zeitraum von 1 bis 600 Sekunden verstanden.

Gegenstand der vorliegenden Erfindung ist ein Verfahren zur, bevorzugt multitonalen, Farbveränderung keratinischer Fasern, das folgende Verfahrensschritte umfasst:

I. Bereitstellen einer Färbe- oder Aufhellzusammensetzung (A), enthaltend

a)i. mindestens ein Alkalisierungsmittel,
a)ii. optional mindestens einen Konsistenzgeber und
a)iii. optional mindestens ein Lösemittel, ausgewählt aus Wasser und mindestens einem organischen Lösemittel, das bevorzugt ausgewählt ist aus einem oder mehreren ein- oder mehrwertigen Alkoholen mit 2 bis 9 Kohlenstoffatomen, Polyethylenglycolen mit 2 bis 20 Ethylenglycoleinheiten sowie Mischungen dieser Lösemittel, und
a)iv. optional mindestens ein Oxidationsfarbstoffvorprodukt und/oder mindestens einen direktziehenden Haarfarbstoff,
a)v. wobei die Färbe- oder Aufhellzusammensetzung (A), sofern sie, bezogen auf ihr Gewicht, mehr als 7 Gew.-% Wasser enthält, einen pH-Wert im Bereich von 6,0 bis 11,5 aufweist, gemessen bei 20°C, und sofern die Zusammensetzung (A), bezogen auf ihr Gewicht, 0 bis 7 Gew.-% Wasser enthält, ihre 30 Gew.-%ige Dispersion in Wasser einen pH-Wert im Bereich von 6,0 bis 12,0, bevorzugt 7,5 - 11,5, besonders bevorzugt 8 bis 11,0, aufweist, jeweils gemessen bei 20°C;

II. Bereitstellen einer Oxidationszusammensetzung (B), enthaltend

b)i. 1 - 18 Gew.-% Wasserstoffperoxid, bezogen auf das Gewicht der Zusammensetzung (B), und
b)ii. Wasser,
b)iii. wobei die Oxidationszusammensetzung (B) einen pH-Wert im Bereich von einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen;

c. Bereitstellen einer Gelierzusammensetzung (C), enthaltend

c)i. 0,1 - 3 Gew.-%, bevorzugt 0,2 - 2 Gew.-%, besonders bevorzugt 0,4 - 1 Gew.-%, bezogen auf das Gewicht der Zusammensetzung (C), mindestens eines Salzes eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, die Gelierzusammensetzung (C) 0,1 - 3 Gew.-%, bevorzugt 0,2 - 2 Gew.-%, besonders bevorzugt 0,4 - 1 Gew.-%, bezogen auf das Gewicht der Zusammensetzung (C), mindestens eines Salzes, ausgewählt aus Kalium-, Calcium-, Strontium-, Barium- und Aluminiumsalzen mit einer Wasserlöslichkeit bei 20°C von mindestens 500 mg/l, enthält,
c)ii. 50 - 99 Gew.-%, bezogen auf das Gewicht der Zusammensetzung (C), Wasser,
c)iii. optional mindestens ein Kationpolymer, das bevorzugt ausgewählt ist aus kationisierten Guar-Ethern;
c)iv. wobei die Gelierzusammensetzung (C) einen pH-Wert im Bereich von 4 bis 12, bevorzugt im Bereich von 4 - 6,5 aufweist, gemessen bei 20°C;

wobei optional die Gelierzusammensetzung (C) *in situ* oder 0,01 bis 24 Stunden vor Durchführung des erfindungsgemäßen Farbveränderungsverfahrens durch Vermischen einer festen, bevorzugt pulverförmigen, Gelierzusammensetzung (C'), enthaltend das mindestens eine unter c)i genannte Salz in Pulverform und optional mindestens

ein Kationpolymer, das bevorzugt ausgewählt ist aus kationisierten Guar-Ethern, in Pulverform, mit Wasser c) iii hergestellt wird, anschließend, das heißt 1 bis 600 Sekunden danach,

d. Herstellen einer Mischung (M) aus (A) und (B), die einen pH-Wert im Bereich von 6,0 bis 11 aufweist, jeweils gemessen bei 20°C; nach 1 bis 600 Sekunden dann

e. Applikation zumindest einer Teilmenge von (M) auf mindestens eine zu färbende und/oder aufzuhellende Keratinfaserpartie;

1 bis 600 Sekunden danach

f. Applizieren, bevorzugt Aufsprühen, der Gelierzusammensetzung (C) auf die mit (M) beaufschlagte(n) Keratinfaserpartie(n);

g. ggf. Wiederholen der Schritte e. und f. im gewünschten Umfang,

h. für eine Zeit von 0,1 bis 60 Minuten, bevorzugt 1 bis 50 Minuten, besonders bevorzugt 10 bis 45 Minuten, außerordentlich bevorzugt 30 bis 45 Minuten Belassen auf den keratinischen Fasern, anschließend Spülen der keratinischen Fasern mit Wasser und ggf. einem Reinigungsmittel, ggf. Nachbehandlung der Fasern mit einem Konditioniermittel und anschließendes Trocknen,

dadurch gekennzeichnet, dass das Verfahren die Applikation von mindestens einem Polysaccharid, das mit Calciumionen in wässrigem Medium ein Gel bilden kann, umfasst, wobei dieses Polysaccharid bevorzugt ausgewählt ist aus Alginsäure, kappa-Carragheenan, iota-Carragheenan und Pektin, und/oder mindestens einem Salz der vorgenannten Polysaccharide, das ausgewählt ist aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist,
wobei diese Polysaccharide nicht in der Gelierzusammensetzung (C) enthalten sind.

Die vorliegende Erfindung bezieht sich auf die oxidative Farbveränderung keratinischer Fasern, insbesondere Haaren. Da bei der Behandlung von keratinischer Fasern, insbesondere Haaren, mit Oxidationsmitteln, insbesondere mit Wasserstoffperoxid, der fasereigene Farbstoff Melanin zu einem gewissen Grad zerstört wird, werden die Fasern/Haare zwangsläufig aufgehellt, ändern also ihre Farbe auch ohne die Gegenwart eines Farbstoffs. Daher umfasst der Begriff "Farbveränderung" im Sinne der vorliegenden Anmeldung sowohl die Aufhellung als auch die Färbung mit einem oder mehreren Farbstoffen.

Für die Farbveränderung von menschlichen Haaren kennt der Fachmann verschiedene Verfahren. Im allgemeinen werden für die Färbung menschlicher Haare entweder direktziehende Farbstoffe oder Oxidationsfarbstoffe verwendet, die durch oxidative Kupplung einer oder mehrerer Entwicklerkomponenten untereinander oder mit einer oder mehreren Kupplerkomponenten entstehen. Kuppler- und Entwicklerkomponenten werden auch als Oxidationsfarbstoffvorprodukte bezeichnet. Die mit Oxidationsfarben erzielten Färbungen werden zumeist als permanente oder semipermanente Färbungen bezeichnet. Als Oxidationsmittel enthalten diese Mittel zumeist Wasserstoffperoxid. Da Wasserstoffperoxid im alkalischen pH-Bereich nur unzureichend lagerstabil ist, umfassen oxidative Färbemittel üblicherweise zwei Komponenten, die unmittelbar, das heißt, in einem Zeitraum von 1 bis 600 Sekunden vor der Anwendung, miteinander vermischt werden. Die eine Komponente, im Folgenden als "Oxidationszusammensetzung (B)" bezeichnet, enthält Wasserstoffperoxid in wässriger Lösung oder Emulsion, wobei diese Zusammensetzung zur Stabilisierung des Wasserstoffperoxids einen sauren pH-Wert im Bereich von 2,5 bis 6,5, bevorzugt im Bereich von 3 - 5,5, besonders bevorzugt im Bereich von 3,5 bis 5,0, aufweist, jeweils bei 20°C gemessen.

Die zweite Komponente, im Folgenden als "Färbe- oder Aufhellzusammensetzung (A)" bezeichnet, enthält ein oder mehrere Alkalisierungsmittel in einer solchen Menge, dass die Anwendungsmischung aus beiden Komponenten einen pH-Wert im Bereich von 8 - 11 aufweist, jeweils bei 20°C gemessen. Sofern die Färbe- oder Aufhellzusammensetzung (A) keinen Farbstoff oder nur geringe Mengen an direktziehenden Farbstoffen enthält - letztere dienen zur Kaschierung unerwünschter Farbtöne, die bei der Melaninoxidation entstehen können -, handelt es sich um ein Aufhell- oder Bleichmittel. Die Färbe- oder Aufhellzusammensetzung (A) kann aber auch Oxidationsfarbstoffvorprodukte und/oder direktziehende Farbstoffe enthalten; die resultierende Anwendungsmischung dient dann als Färbemittel. Daneben gibt es auch Färbekits und Färbeverfahren, bei denen die Anwendungsmischung aus beiden Komponenten einen pH-Wert im Bereich von etwa 6 bis 7,9 aufweist; die Färbeergebnisse dieser so genannten "sauren" Färbungen erreichen allerdings häufig nicht die Güte, die mit stärker alkalischen Anwendungsmischungen erzielt werden. Für das Färbe- oder Aufhellergebnis ist es wichtig, dass das anwendungsbereite Färbe- oder Aufhellmittel, das durch Vermischen der erfindungsgemäß verwendeten Färbe- oder Aufhellzusammensetzung (A) mit der Zusammensetzung (B) erhalten wird, einen pH-Wert im Bereich von 6,5 bis 11,0, bevorzugt 8 bis 10,5, besonders bevorzugt 8,5 bis 9,5, aufweist, jeweils gemessen bei 20°C. Bei diesen pH-Werten öffnet sich die äußere Keratinfaserschicht optimal zur Aufnahme der Oxidationsfarbstoffvorprodukte und/oder entfaltet sich die oxidative Wirkung des Wasserstoffperoxids und ggf. weiterer Peroxidverbindungen optimal.

Polysaccharid, ausgewählt aus Alginsäure, kappa-Carragheenan, iota-Carragheenan und Pektin, und/oder mindestens ein Salz dieser Polysaccharide

Ein wesentlicher Bestandteil erfindungsgemäßer und erfindungsgemäß bevorzugter Färbeverfahren ist mindestens ein Polysaccharid, das mit Calciumionen in wässrigem Medium ein Gel bildet. Erfindungsgemäß bevorzugte Polysaccharide, die mit Calciumionen in wässrigem Medium ein Gel bilden können, sind ausgewählt aus Alginsäure, kappa-Carragheenan, iota-Carragheenan und Pektin sowie mindestens einem Salz der vorgenannten Polysaccharide, das ausgewählt aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist. Wässrige Lösungen dieser Polysaccharide und Polysaccharidsalze zeigen je nach Konzentration eine nur leicht erhöhte Viskosität, bilden aber noch keine festen Gele.

Besonders bevorzugt sind Alginsäure, Natriumalginat, Ammoniumalginat, Magnesiumalginat, Monoethanolammoniumalginat, kappa-Carragheenan, das Lithiumsalz von kappa-Carragheenan, das Natriumsalz von kappa-Carragheenan, iota-Carragheenan, das Lithiumsalz von iota-Carragheenan, das Natriumsalz von iota-Carragheenan, das Kaliumsalz von iota-Carragheenan, das Ammoniumsalz von iota-Carragheenan, Pektin, Natriumpektinat, Ammoniumpektinat, Kaliumpektinat und Magnesiumpektinat, sowie Mischungen dieser Substanzen. Erfindungsgemäß außerordentlich bevorzugt sind Mischungen aus Alginsäure und Natriumalginat, insbesondere Mischungen aus Alginsäure und Natriumalginat im Gewichtsverhältnis von Alginsäure zu Natriumalginat im Bereich von 1:2 bis 2:1, bevorzugt im Bereich von 0,8 bis 1,25, besonders bevorzugt im Gewichtsverhältnis 1:1.

Die vorstehend genannten Polysaccharide und/oder die genannten Salze zeigen ein weitgehend pHunabhängiges Verdickungsverhalten. Allerdings gelieren sie bei Zugabe bestimmter mehrwertiger Ionen, wie Calcium-, Strontium-, Barium- und Aluminiumionen. Darüber hinaus geliert kappa-Carragheenan auch bei Zugabe von Kaliumionen.

Dieses Verhalten wird im Sinne der vorliegenden Anmeldung dazu genutzt, auf ausgewählten Keratinfaserpartien oder Keratinfasersträhnen nach der Applikation des Färbe- oder Aufhellmittels gezielt einen Folien-ähnlichen Film zu erzeugen, der einen Austausch mit dem auf benachbarten Strähnen applizierten Färbe- oder Aufhellmittel auch bei direktem Kontakt verhindert oder zumindest stark reduziert. Um diesen Film zu erzeugen, gibt es zwei Ausführungsformen des erfindungsgemäßen Verfahrens.

In der ersten bevorzugten Ausführungsform des erfindungsgemäßen Verfahrens ist mindestens eines der vorstehend genannten Polysaccharide und/oder mindestens eines der vorstehend genannten Salze der Polysaccharide in einer der Zusammensetzungen (A) oder (B) oder in beiden Zusammensetzungen (A) und (B) enthalten und/oder wird der anwendungsbereiten Färbe- oder Aufhellmittelmischung aus (A) und (B) über eine dritte Komponente) zugemischt. Da die vorstehend genannten Polysaccharide oder ihre genannten Salze im stärker alkalischen pH-Bereich nicht so stark verdicken wie beispielsweise (Meth)Acrylsäure-, (Meth)Acrylsäureester-, (Meth)Acrylamid- und/oder 2-Acrylamido-2-methylpropansulfonsäure-Polymere, wie sie in dem Färbeverfahren gemäß WO2014164213A1 zum Einsatz kommen oder wie ionische Assoziativpolymere, die in dem Färbeverfahren gemäß EP2574331A2 zum Einsatz kommen, weist das Polysaccharid-haltige Färbemittel nur eine mittlere Viskosität im Bereich von etwa 2000 bis 6000 mPas (20°C) auf und lässt sich so leicht und gleichmäßig auf den Keratinfasern verteilen. Erst wenn die Keratinfasersträhnen ausreichend mit Färbe- oder Aufhellmittel beaufschlagt sind, wird eine Gelierzusammensetzung appliziert, die Calcium-, Strontium-, Barium- oder/und Aluminiumionen oder, falls das Polysaccharid aus kappa-Carragheenan ausgewählt ist, Kaliumionen, Calcium-, Strontium-, Barium- oder/und Aluminiumionen in Form wasserlöslicher Salze mit einer Wasserlöslichkeit von mindestens 500 mg/l in wässriger Lösung enthält. Die Gelierzusammensetzung (C) kann zum Beispiel mit einem Pinsel oder einem vergleichbaren Applikator auf die Keratinfasern aufgetragen oder aber aufgesprüht werden. Durch die Fällungsreaktion zwischen dem Polysaccharid oder seinem löslichen Salz und den Gel bildenden Kationen aus der Gelierzusammensetzung (C) bildet sich in situ auf den mit dem Färbe-oder Aufhellmittel beaufschlagten Keratinfasern ein filmförmiges Gel aus, das als Trennschicht analog zu einer Strähnchenfolie fungiert.

In einer zweiten, besonders bevorzugten, Ausführungsform des erfindungsgemäßen Verfahrens liegt mindestens eines der vorstehend genannten Polysaccharide und/oder mindestens eines der vorstehend genannten Salze der Polysaccharide in Form einer wässrigen Lösung oder Dispersion vor und wird, ähnlich wie die vorstehend erläuterte Gelierzusammensetzung (C), auf die ausreichend mit Färbe- oder Aufhellmittel beaufschlagten Keratinfasersträhnen aufgetragen. Dies kann ebenfalls mit einem Pinsel oder einem vergleichbaren Applikator, aber auch durch Aufsprühen auf die Keratinfasern erfolgen. Bevorzugt wird erst die wässrige Zubereitung des Polysaccharids, das mit Calciumionen gelieren kann, appliziert. Anschließend wird Gelierzusammensetzung (C) aufgetragen.

In einer dritten spezifischen Ausführungsform des erfindungsgemäßen Verfahrens liegt mindestens eines der vorstehend genannten Polysaccharide und/oder mindestens eines der vorstehend genannten Salze der Polysaccharide in Form einer wässrigen Lösung oder Dispersion vor. Zunächst wird die vorstehend erläuterte Gelierzusammensetzung (C) auf die ausreichend mit Färbe- oder Aufhellmittel beaufschlagten Keratinfasersträhnen aufgetragen. Dies kann mit einem Pinsel oder einem vergleichbaren Applikator, aber auch durch Aufsprühen auf die Keratinfasern erfolgen. Anschließend wird die wässrige Zubereitung des Polysaccharids, das mit Calciumionen gelieren kann, appliziert.

Wenn nach Ablauf der empfohlenen Einwirkzeit die Färbe- oder Aufhellreaktion beendet ist, kann das gesamte Haarbehandlungsmittel in einem einzigen Arbeitsgang mit Wasser und optional einem Shampoo ausgewaschen werden. Das zeitraubende Abwickeln, wie es bei bekannten Strähnchenfolien nötig ist, entfällt vorliegend. Da die erfindungsgemäß verwendeten Polysaccharide natürlich vorkommende Biopolymere darstellen, sind sie ohne Probleme in Kläranlagen biologisch abbaubar. Sie sind also deutlich einfacher zu entsorgen als flächenförmige Folienmaterialien.

Färbe- oder Aufhellzusammensetzung (A)

Die Färbe-oder Aufhellzusammensetzung (A) kann fest, insbesondere pulverförmig, sein, aber auch flüssig (0 bis 500 mPas bei 20°C), mittelviskos (> 400 bis 4000 mPas bei 20°C), cremeförmig (> 4000 bis 40.000 mPas bei 20 °C) oder pastenförmig (> 40.000 bis 4.000.000 mPas bei 20°C). Feste Färbe- oder Aufhellzusammensetzungen (A) können auch in Tablettenform oder granuliert vorliegen.

Aufhellzusammensetzunqen (A-1) und (A-2)

In einer ersten bevorzugten Ausführungsform der Erfindung ist die Zusammensetzung (A) ein Mittel, das nach Vermischen mit einer Wasserstoffperoxid-haltigen wässrigen Zusammensetzung (B) zur Aufhellung und/oder Blondierung von Keratinfasern, insbesondere von Humanhaar, dient.

In einer ersten besonders bevorzugten Ausführungsform der Erfindung ist die Aufhellzusammensetzung (A-1) flüssig (0 bis 500 mPas bei 20°C), mittelviskos (> 400 bis 4000 mPas bei 20°C) oder cremeförmig (> 4000 bis 40.000 mPas bei 20 °C) und enthält Wasser in einer Menge von mehr als 7 Gew.-% bis 90 Gew.-%, bevorzugt 20 bis 85 Gew.-%, besonders bevorzugt 40 bis 75 Gew.-%, jeweils bezogen auf das Gewicht der Aufhellzusammensetzung (A-1).

In einer zweiten besonders bevorzugten Ausführungsform der Erfindung ist die Aufhellzusammensetzung (A-2) fest, insbesondere pulverförmig, kann aber auch in Tablettenform oder granuliert vorliegen, und enthält 0 bis weniger als 7 Gew.-% Wasser, bezogen auf das Gewicht der jeweiligen Aufhellzusammensetzung (A-2).

Färbezusammensetzungen (A-3) und (A-4)

In einer weiteren besonders bevorzugten Ausführungsform der Erfindung ist die Zusammensetzung (A) ein Mittel, das nach Vermischen mit einer Wasserstoffperoxid-haltigen wässrigen Zusammensetzung (B) zur Färbung von Keratinfasern, insbesondere von Humanhaar, dient.

In einer dritten besonders bevorzugten Ausführungsform der Erfindung ist die Färbezusammensetzung (A-3) flüssig (0 bis 500 mPas bei 20°C), mittelviskos (> 400 bis 4000 mPas bei 20°C) oder cremeförmig (> 4000 bis 40.000 mPas bei 20 °C) und enthält Wasser in einer Menge von mehr als 7 Gew.-% bis 90 Gew.-%, bevorzugt 20 bis 85 Gew.-%, besonders bevorzugt 40 bis 75 Gew.-%, jeweils bezogen auf das Gewicht der Färbezusammensetzung (A-3), sowie weiterhin mindestens ein Oxidationsfarbstoffvorprodukt.

In einer vierten besonders bevorzugten Ausführungsform der Erfindung ist die Färbezusammensetzung (A-4) fest, insbesondere pulverförmig, kann aber auch in Tablettenform oder granuliert vorliegen und enthält 0 bis weniger als 7 Gew.-% Wasser, bezogen auf das Gewicht der jeweiligen Färbezusammensetzung (A-4), sowie weiterhin mindestens ein Oxidationsfarbstoffvorprodukt.

pH-Wert der Färbe- oder Aufhellzusammensetzungen (A-1) und (A-3)

Die Färbe- oder Aufhellzusammensetzungen (A-1) und (A-3) weisen einen pH-Wert im Bereich von 6,5 bis 12,0, bevorzugt 8 - 11,5, besonders bevorzugt 8,5 bis 11,0, auf, jeweils gemessen bei 20°C.

Die Färbe- oder Aufhellzusammensetzungen (A-2) und (A-4) liegen fest, bevorzugt pulverförmig, oder pastenförmig vor und enthalten kein oder nur bis ca. 7 Gew.-% Wasser. Daneben enthalten sie so viel Alkalisierungsmittel, dass ihre 30 Gew.-%ige Dispersion in Wasser einen pH-Wert im Bereich von 6,0 bis 12,0, bevorzugt 7,5 - 11,5, besonders bevorzugt 8 bis 11,0, aufweist, jeweils gemessen bei 20°C.

Alkalisierungsmittel

Die Färbe- oder Aufhellzusammensetzungen (A), nämlich (A-1), (A-2), (A-3) und (A-4), enthalten mindestens ein Alkalisierungsmittel, das ausgewählt ist aus Ammoniumhydroxid, Monoethanolamin und Natriumsilikaten sowie Mischungen hiervon.

Bevorzugt enthalten die Aufhellzusammensetzung (A-1) und die Färbezusammensetzung (A-3) Ammoniumhydroxid, also Ammoniak in Form seiner wässrigen Lösung. Bei entsprechenden wässrigen Ammoniak-Lösungen kann es sich um 10 bis 35 prozentige Lösungen handeln (berechnet in Gew.-%, 100 g wässrige Ammoniaklösung enthalten

dementsprechend 10 bis 35 g Ammoniak). Bevorzugt wird Ammoniak in Form einer 20 bis 30 Gew.-%igen Lösung, besonders bevorzugt in Form einer 25 Gew.-%igen Lösung eingesetzt.

In einer besonders bevorzugten Ausführungsform sind die erfindungsgemäß verwendeten Zusammensetzungen (A-1) und (A-3) dadurch gekennzeichnet, dass sie Ammoniumhydroxid in einer Menge von 0,20 bis 18,0 Gew.-%, bevorzugt von 1,0 bis 15 Gew.-%, weiter bevorzugt von 2,0 bis 12,0 Gew.-% und besonders bevorzugt von 3,0 bis 9 Gew.-% - bezogen auf das Gewicht der erfindungsgemäß verwendeten Zusammensetzung (A) - enthalten.

Zusätzlich zu bzw. anstelle von Ammoniumhydroxid enthalten bevorzugte erfindungsgemäß verwendete Zusammensetzungen (A), insbesondere die erfindungsgemäß verwendeten Zusammensetzungen (A-1) und (A-3), Monoethanolamin.

Zur Erzielung einer maximalen Geruchsminimierung und zur Optimierung der Echtheitseigenschaften ist Monoethanolamin in einer Gesamtmenge von 0,2 - 9,0 Gew.-%, bevorzugt von 1,0 - 7 Gew.-%, weiter bevorzugt von 2,0 bis 6,0 Gew.-% und besonders bevorzugt von 3,0 bis 5,5 Gew.-% - bezogen auf das Gewicht der erfindungsgemäß verwendeten Zusammensetzung (A) - enthalten. Zur Erzielung einer maximalen Geruchsminimierung und zur Optimierung der Echtheitseigenschaften enthalten die erfindungsgemäß verwendeten Zusammensetzungen (A-1) und (A-3) Monoethanolamin in einer Gesamtmenge von 0,2 - 9,0 Gew.-%, bevorzugt von 1,0 - 7 Gew.-%, weiter bevorzugt von 2,0 bis 6,0 Gew.-% und besonders bevorzugt von 3,0 bis 5,5 Gew.-%, jeweils bezogen auf das Gewicht der erfindungsgemäß verwendeten Zusammensetzungen (A-1) oder (A-3).

Bei Natriumsilikaten im Sinne der vorliegenden Erfindung handelt es sich um chemische Verbindungen, die sich aus Natriumoxid und Siliciumdioxid zusammensetzen und die in verschiedenen molaren Verhältnissen (Monosilikat, Metasilikat und Polysilikat) vorkommen können. Ein Beispiel für ein Natriumsilikat ist das Natriumsalz der Orthokieselsäure mit der Summenformel $Na_4SiO_4$, das auch als Natriumorthosilikat bezeichnet wird.

Weitere Beispiele für geeignete Natriumsilikate sind das Dinatriummetasilikat bzw. Natriummetasilikat mit der Summenformel $Na_2SiO_3$, das Dinatriumdisilikat mit der Summenformel $Na_2Si_2O_5$ oder das Dinatriumtrisilikat mit der Summenformel $Na_2Si_3O_7$.

Silikate in amorpher Form können durch das Zusammenschmelzen von Siliciumdioxid und Alkalioxid in molaren Verhältnissen zwischen 1:1 und 4:1 hergestellt werden. Die so erhaltenen Feststoffe werden bei etwa 150 °C und 5 bar Dampfdruck gelöst, um eine Lösung der Natriumsilikate in Wasser zu erhalten, bei diesen entsprechenden Lösungen handelt es sich um Alkaliwassergläser.

Als Alkaliwassergläser werden aus einer Schmelze erstarrte, glasartige (amorphe) Natriumsilikate oder ihre wässrigen Lösungen bezeichnet. Diese nennt man auch Natronwasserglas. Auch Natronwassergläser sind innerhalb dieser Erfindung von der Definition der Natriumsilikate umfasst.

Die molare Zusammensetzung bei Wassergläsern beträgt üblicherweise 2 bis 4 mol $SiO_2$ auf 1 mol Alkalioxid ($Na_2O$).

Ein Beispiel für ein bevorzugtes Natriumsilikat ist Natronwasserglas, das in Form seiner wässrigen Lösung vorliegt, einen $Na_2O$-Gehalt von 7,5 bis 8,8 Gew.-% und einen SiO2 Gehalt von 25,0 bis 28,5 Gew.-% besitzt und das die CAS-Nr. 1344-09-5 (Chemical Abstracts Number) hat.

Weitere erfindungsgemäß bevorzugt verwendete Zusammensetzungen (A) enthalten als Alkalisierungsmittel mindestens ein Natriumsilikat, bevorzugt Natriummetasilikat oder Natronwasserglas, in einer Gesamtmenge von 0,1 bis 25 Gew.-%, bevorzugt 0,5 bis 20 Gew.-%, besonders bevorzugt 1 bis 17 Gew.-%, außerordentlich bevorzugt 2 bis 9 Gew.-%, jeweils bezogen auf das Gewicht der erfindungsgemäß verwendeten Zusammensetzung (A).

Der Einsatz von Natriumsilikat als Alkalisierungsmittel ist insbesondere für die erfindungsgemäß verwendeten Zusammensetzungen (A-2) und (A-4) bevorzugt.

Weitere erfindungsgemäß bevorzugt verwendete Zusammensetzungen (A-2) und (A-4) enthalten als Alkalisierungsmittel mindestens ein Natriumsilikat, bevorzugt Natriummetasilikat oder Natronwasserglas, in einer Gesamtmenge von 0,1 bis 25 Gew.-%, bevorzugt 0,5 bis 20 Gew.-%, besonders bevorzugt 1 bis 17 Gew.-%, außerordentlich bevorzugt 2 bis 9 Gew.-%, jeweils bezogen auf das Gewicht der erfindungsgemäß verwendeten Zusammensetzung (A-2) oder (A-4).

Weitere erfindungsgemäß bevorzugt verwendete Zusammensetzungen (A), insbesondere die Zusammensetzungen (A-2) und (A-4), enthalten als Alkalisierungsmittel mindestens ein Alkalimetall- oder Erdalkalimetallcarbonat, das bevorzugt ausgewählt ist aus Natriumcarbonat, Kaliumcarbonat und Magnesiumcarbonat, sowie Mischungen dieser Carbonate. Das mindestens eine Alkalimetall- oder Erdalkalimetallcarbonat ist bevorzugt in einer Gesamtmenge von 0,1 bis 25 Gew.-%, bevorzugt 0,5 bis 20 Gew.-%, besonders bevorzugt 1 bis 10 Gew.-%, enthalten, jeweils bezogen auf das Gewicht der erfindungsgemäß verwendeten Zusammensetzung (A). Das mindestens eine Alkalimetall- oder Erdalkalimetallcarbonat ist bevorzugt in einer Gesamtmenge von 0,1 bis 25 Gew.-%, bevorzugt 0,5 bis 20 Gew.-%, besonders bevorzugt 1 bis 10 Gew.-%, enthalten, jeweils bezogen auf das Gewicht der erfindungsgemäß verwendeten Zusammensetzung (A-2) oder (A-4).

Weiterhin können andere Alkalisierungsmittel, wie Kaliumhydroxid (KOH) und Natriumhydroxid (NaOH) enthalten sein, meist in einer Gesamtmenge von 0,05 bis 1,5 Gew.-%, bevorzugt 0,1 bis 0,6 Gew.-%, jeweils bezogen auf

das Gewicht der erfindungsgemäß verwendeten Zusammensetzung (A).

Auch basische Aminosäuren, insbesondere Arginin, Lysin oder Histidin sowie Mischungen dieser Aminosäuren, insbesondere Mischungen aus Arginin und Lysin, können in bevorzugt verwendeten Zusammensetzungen (A) als Alkalisierungsmittel enthalten sein, bevorzugt in einer Gesamtmenge von 0,01 bis 10 Gew.-%, bevorzugt 0,1 bis 5 Gew.-%, besonders bevorzugt 0,5 bis 3 Gew.-%, jeweils bezogen auf das Gewicht der erfindungsgemäß verwendeten Zusammensetzung (A). Erfindungsgemäß besonders bevorzugt verwendete Zusammensetzungen (A) enthalten Mischungen der vorgenannten Alkalisierungsmittel, insbesondere Ammoniumhydroxid/Natriumsilikat/Kaliumhydroxid-Mischungen, Ammoniumhydroxid/Monoethanolamin/Natriumsilikat/Kaliumhydroxid-Mischungen, Monoethanolamin/ Natriumsilikat/Kaliumhydroxid-Mischungen, Ammoniumhydroxid/Natriumsilikat/Kaliumhydroxid/Arginin-Mischungen, Ammoniumhydroxid/Monoethanolamin/Natriumsilikat/Kaliumhydroxid/Arginin-Mischungen, Monoethanolamin/Natriumsilikat/Kaliumhydroxid/Arginin-Mischungen, Natriumsilikat/Magnesiumcarbonat-Mischungen und Natriummetasilikat/Magnesiumcarbonat-Mischungen.

Konsistenzgeber

Erfindungsgemäß bevorzugt verwendete Zusammensetzungen (A), insbesondere die Zusammensetzungen (A-1) und (A-3), enthalten mindestens einen Konsistenzgeber. Geeignete Konsistenzgeber sind insbesondere Fettsubstanzen mit einem Schmelzpunkt von 25 °C oder darüber bei 1013 mbar Umgebungsdruck sowie polymere Verdicker, die von den erfindungsgemäß verwendeten Polysacchariden, die mit Calciumionen in wässrigem Medium ein Gel bilden, verschieden sind. Derartige Konsistenzgeber sind bevorzugt ausgewählt aus linearen gesättigten 1-Alkanolen mit 12 - 30 Kohlenstoffatomen und Glycerylfettsäureestern der allgemeinen Formel (I),

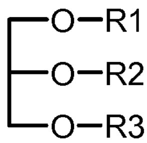
(I),

worin
R1, R2 und R3 unabhängig voneinander für ein Wasserstoffatom oder eine Gruppierung der Formel (II) stehen,

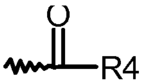
(II),

worin
R4 für eine unverzweigte oder verzweigte, gesättigte oder ungesättigte $C_{11}$-$C_{27}$-Alkylgruppe steht, mit der Maßgabe, dass mindestens einer und maximal zwei der Reste ausgewählt aus R1, R2 und R3 für eine Gruppierung der Formel (II) stehen.

Der Rest R4 in der Formel (II) steht für eine unverzweigte oder verzweigte, gesättigte oder ungesättigte $C_{11}$-$C_{27}$-Alkylgruppe.

Bevorzugt steht R4 für eine unverzweigte, gesättigte $C_{11}$-$C_{27}$-Alkylgruppe. Weiter bevorzugt steht R4 für eine unverzweigte, gesättigte $C_{13}$-$C_{23}$-Alkylgruppe. Besonders bevorzugt steht R4 für eine unverzweigte, gesättigte $C_{15}$-$C_{17}$-Alkylgruppe.

Erfindungsgemäß besonders bevorzugte Glycerylfettsäureester der allgemeinen Formel (I) sind ausgewählt aus mindestens einer Verbindung aus der Gruppe der Formeln (Ia) bis (Id):

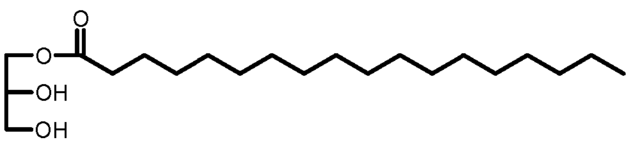
(Ia)

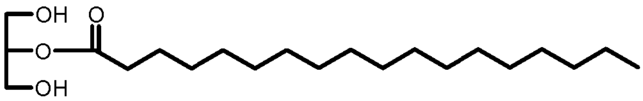

(Ib)

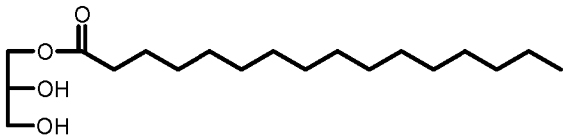

(Ic)

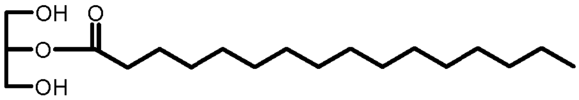

(Id)

Die Verbindungen der Formeln (Ia) bis (Id) sind auch unter den Namen Glycerylmonostearat und Glycerylmonopalmitat bekannt.

Weitere erfindungsgemäß bevorzugt verwendete Färbe- oder Aufhellzusammensetzungen (A) sind dadurch gekennzeichnet, dass als Glycerylfettsäureester der allgemeinen Formel (I) mindestens eine Verbindung aus der Gruppe der Formeln (Ie) bis (Ih) enthalten ist:

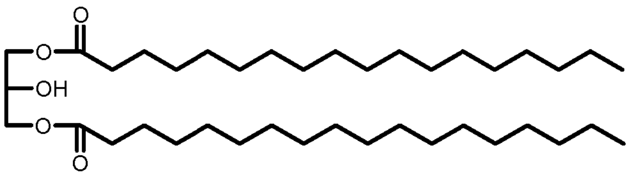

(Ie)

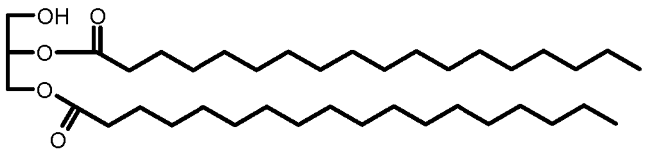

(If)

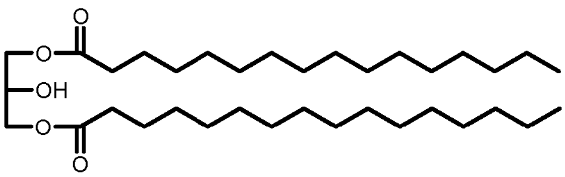

(Ig)

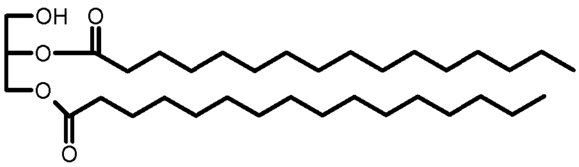

(Ih)

Die Verbindungen der Formel (Ie) bis (Ih) sind auch unter den Namen Glyceryldistearat und Glyceryldipalmitat bekannt.

Erfindungsgemäß bevorzugte lineare gesättigte 1-Alkanole mit 12 - 30 Kohlenstoffatomen sind ausgewählt aus

Laurylalkohol, Myristylalkohol, Cetylalkohol, Stearylalkohol, Arachidylalkohol und Behenylalkohol sowie aus Mischungen dieser Alkanole, besonders bevorzugt aus Cetylalkohol, Stearylalkohol, Arachidylalkohol, Behenylalkohol und Cetylalkohol/ Stearylalkohol-Mischungen. Weitere erfindungsgemäß bevorzugte Konsistenzgeber sind Ethylenglycolmonostearat, Ethylenglycoldistearat, Wachse, Glycerintriester, die einen Schmelzpunkt von 25 °C oder darüber aufweisen, insbesondere hydriertes Rizinusöl (Castorwachs) und ethoxylierte hydrierte Rizinusöle, und Fettsäure-Fettalkoholester mit einem Schmelzpunkt von 25 °C oder darüber, sowie Mischungen dieser Substanzen.

Erfindungsgemäß bevorzugt verwendete Zusammensetzungen (A), insbesondere die Zusammensetzungen (A-1) und (A-3), enthalten mindestens einen Fettsubstanz-Konsistenzgeber in einer Gesamtmenge von 0,1 bis 30 Gew.-%, bevorzugt 0,5 bis 20 Gew.-%, besonders bevorzugt 2 bis 15 Gew.-%, außerordentlich bevorzugt 3 bis 10 Gew.-%, jeweils bezogen auf das Gewicht der Zusammensetzung (A). Weitere bevorzugte verwendete Zusammensetzungen (A), insbesondere die Zusammensetzungen (A-1) und (A-3), enthalten, jeweils bezogen auf ihr Gewicht, mindestens ein lineares gesättigtes 1-Alkanol mit 12 - 30 Kohlenstoffatomen in einer Gesamtmenge von 0,1 bis 30 Gew.-%, bevorzugt 0,5 bis 20 Gew.-%, besonders bevorzugt 2 bis 15 Gew.-%, außerordentlich bevorzugt 3 bis 10 Gew.-%, jeweils bezogen auf das Gewicht der Zusammensetzung (A).

In einer besonders bevorzugten Ausführungsform der Erfindung sind die erfindungsgemäß verwendeten Zusammensetzungen (A), (B), (C) und (D) frei von anionischen oder kationischen Assoziativpolymeren, die mit ggf. ethoxylierten, $C_6$ - $C_{24}$-Alkyl- oder $C_6$ - $C_{24}$-Alkenyl-Gruppen substituiert sind. In einer außerordentlich bevorzugten Ausführungsform der Erfindung sind die erfindungsgemäß verwendeten Zusammensetzungen (A), (B), (C) und (D) frei von anionischen oder kationischen Assoziativpolymeren, die mit ggf. ethoxylierten, $C_6$ - $C_{24}$-Alkyl- oder $C_6$ - $C_{24}$-Alkenyl-Gruppen substituiert sind und ausgewählt sind aus Acrylates/Ceteth-20 Itaconate Copolymeren, Polyurethane-39-Polymeren, Acrylates/Beheneth-25 Methacrylate Copolymeren und Acrylates/C10-30 Alkyl Acrylate Crosspolymeren.

Polysaccharid in Zusammensetzung (A)

Sofern das mindestens eine Polysaccharid, das mit Calciumionen im wässrigen Medium gelieren kann und das bevorzugt ausgewählt ist aus Alginsäure, kappa-Carragheenan, iota-Carragheenan und Pektin, und/oder mindestens einem Salz der vorgenannten Polysaccharide, das ausgewählt ist aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist, in der Färbe- oder Aufhellzusammensetzung (A) enthalten ist, liegt es in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (A), vor. Besonders bevorzugt enthält die Färbe- oder Aufhellzusammensetzung (A) eine Mischung aus Alginsäure und Natriumalginat in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (A), wobei es bevorzugt ist, wenn das Gewichtsverhältnis von Alginsäure zu Natriumalginat im Bereich von 1:2 bis 2:1, bevorzugt im Bereich von 0,8 bis 1,25, besonders bevorzugt im Gewichtsverhältnis 1:1 liegt.

Oxidationsfarbstoffvorprodukt und/oder direktziehender Haarfarbstoff

Wenn die Mischung aus erfindungsgemäß verwendeter Zusammensetzung (A) und Zusammensetzung (B) zum oxidativen Färben von Keratinfasern, insbesondere von Humanhaar, bestimmt ist, enthält die Zusammensetzung (A) zur Ausbildung der Farbstoffe mindestens ein Oxidationsfarbstoffvorprodukt.

Unter die Oxidationsfarbstoffvorprodukte fallen Oxidationsfarbstoffvorprodukte vom Entwickler-Typ und vom Kuppler-Typ. Besonders geeignete Oxidationsfarbstoffvorprodukte vom Entwickler-Typ sind dabei ausgewählt aus mindestens einer Verbindung aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind ausgewählt aus der Gruppe 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen, insbesondere den Sulfaten, Hydrogensulfaten, Chloriden und Hydrochloriden.

Außerordentlich bevorzugt sind 4,5-Diamino-1-(2-hydroxyethyl)pyrazol, 4-Amino-3-methylphenol, 4-Amino-2-Hydroxytoluen und m-Aminophenol sowie Mischungen dieser Farbstoffvorprodukte.

In einer bevorzugten Ausführungsform enthalten die erfindungsgemäß verwendeten Zusammensetzungen (A) ein oder mehrere Oxidationsfarbstoffvorprodukte in einer Gesamtmenge von 0,01 bis 30,0 Gew.-%, bevorzugt von 0,1 bis 15 Gew.-%, weiter bevorzugt von 0,6 bis 3,1 Gew.-% und ganz besonders bevorzugt von 1,2 bis 2,2 Gew.-%, bezogen auf das Gewicht der erfindungsgemäß verwendeten Zusammensetzung (A).

In einer weiteren bevorzugten Ausführungsform enthalten die erfindungsgemäß verwendeten Zusammensetzungen (A) mindestens einen direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Bevorzugt ist mindestens ein direktziehender Farbstoff in einer Gesamtmenge von 0,001 bis 3 Gew.-%, bezogen auf das Gewicht der erfindungsgemäß verwendeten Zusammensetzung (A) enthalten. Direktziehende Farbstoffe dienen in oxidativen Färbemitteln (A-3) und (A-4) zur Nuancierung des erzielten Farbtons, in oxidativen Blondiermitteln (A-1) und (A-2) zum Ausgleich unerwünschter Rot- und Orangetöne, die beim Abbau des haareigenen Melanins entstehen können.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Tetrabromphenolblau, Acid Red 33 und Acid Red 52 sowie deren Mischungen sind für Blondiermittel (A-1) und (A-2) erfindungsgemäß besonders bevorzugt.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterozyklus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

## Oxidationszusammensetzung (B)

### Wasserstoffperoxid

Die Ausbildung der Farbstoffe in oxidativen Färbemitteln bzw. der Abbau des haareigenen Farbstoffs Melanin zur Blondierung erfolgt erst durch den Einfluss einer Peroxidverbindung als Oxidationsmittel. Üblicherweise wird hierfür Wasserstoffperoxid verwendet. Der Einsatz von Wasserstoffperoxid kann nur in Form einer wässrigen Lösung erfolgen.

Erfindungsgemäß bevorzugt verwendete Oxidationszusammensetzungen (B) sind dadurch gekennzeichnet, dass sie 0,5 bis 13 Gew.-%, weiter bevorzugt 1 bis 9 Gew.-%, besonders bevorzugt 2 bis 7 Gew.-% und ganz besonders bevorzugt 3 bis 4,5 Gew.-% Wasserstoffperoxid (berechnet als 100 %-iges $H_2O_2$) enthalten, jeweils bezogen auf das

Gesamtgewicht der Oxidationszusammensetzung (B).

Blondiermittel (A-2) oder Färbemittel (A-4), die besonders stark aufhellen sollen, zum Beispiel zur Färbung von sehr dunklen, Melanin-reichen Keratinfasern, können darüber hinaus stark oxidierende Peroxidverbindungen, wie Kalium-, Natrium- und/oder Ammoniumpersulfat und/oder Natriumpercarbonat enthalten.

Wasser

Die erfindungsgemäß verwendeten Oxidationszusammensetzungen (B) enthalten Wasser, und zwar bevorzugt in einer Menge von 20 bis 95 Gew.-%, bevorzugt 30 bis 90 Gew.-%, besonders bevorzugt 40 bis 85 Gew.-%, außerordentlich bevorzugt 50 bis 80 Gew.-%, jeweils bezogen auf das Gesamtgewicht der Oxidationszusammensetzung (B).

pH-Wert

Erfindungsgemäß verwendete Oxidationszusammensetzungen (B) weisen einen pH-Wert im Bereich von 2,5 - 6,5, bevorzugt im Bereich von 3 - 5,5, besonders bevorzugt im Bereich von 3,5 bis 5,0, auf, jeweils bei 20°C gemessen.

Polysaccharid in der Oxidationszusammensetzung (B)

Sofern das mindestens eine Polysaccharid, das mit Calciumionen im wässrigen Medium gelieren kann und das bevorzugt ausgewählt ist aus Alginsäure, kappa-Carragheenan, iota-Carragheenan und Pektin, und/oder mindestens einem Salz der vorgenannten Polysaccharide, das ausgewählt ist aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist, in der Oxidationszusammensetzung (B) enthalten ist, liegt es in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (B), vor. Besonders bevorzugt enthält die Oxidationszusammensetzung (B) eine Mischung aus Alginsäure und Natriumalginat in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (B), wobei es bevorzugt ist, wenn das Gewichtsverhältnis von Alginsäure zu Natriumalginat im Bereich von 1:2 bis 2:1, bevorzugt im Bereich von 0,8 bis 1,25, besonders bevorzugt im Gewichtsverhältnis 1:1 liegt.

Organische Lösemittel

Weiterhin können alle erfindungsgemäß verwendeten Zusammensetzungen, insbesondere die Oxidationszusammensetzungen (B) und die Färbe- oder Aufhellzusammensetzungen (A-1) und (A-3), weniger bevorzugt auch (A-2) und (A-4), mindestens ein organisches Lösemittel enthalten, das bevorzugt ausgewählt ist aus einem oder mehreren ein- oder mehrwertigen Alkoholen mit 2 bis 9 Kohlenstoffatomen, Polyethylenglycolen mit 2 bis 20 Ethylenglycoleinheiten sowie Mischungen dieser Lösemittel, bevorzugt in einer Gesamtmenge von 0,001 bis 80 Gew.-%, besonders bevorzugt 0,01 bis 50 Gew.-%, außerordentlich bevorzugt 0,5 bis 20 Gew.-%, weiter außerordentlich bevorzugt 1,5 bis 10 Gew.-%, jeweils bezogen auf das Gewicht der jeweiligen erfindungsgemäß verwendeten Zusammensetzung. Bevorzugte organische Lösemittel sind ausgewählt aus $C_1$-$C_4$-Alkoholen, insbesondere Ethanol und Isopropanol, weiterhin ausgewählt aus mehrwertigen Alkoholen mit 2 bis 9, bevorzugt ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 2-Ethyl-2-hydroxymethyl-1,3-propandiol, Dipropylenglycol, Tripropylenglycol, Diglycerin und Triglycerin sowie weiterhin ausgewählt aus Polyethylenglycolen mit 2 bis 20 Ethylenglycoleinheiten, insbesondere PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Die Verwendung von Mischungen der vorgenannten Substanzen ist erfindungsgemäß ebenfalls bevorzugt.

Mischungsverhältnisse (A) (B)

Weiterhin ist es für das Färbe- oder Aufhellergebnis wichtig, dass das anwendungsbereite Färbe-oder Aufhellmittel, das durch Vermischen einer erfindungsgemäß verwendeten Färbe- oder Aufhellzusammensetzung (A) mit einer erfindungsgemäß verwendeten Oxidationszusammensetzung (B) erhalten wird, einen pH-Wert im Bereich von 6,5 bis 11,0, bevorzugt 8 bis 10,5, besonders bevorzugt 8,5 bis 9,5, aufweist, jeweils gemessen bei 20°C. Bei diesen pH-Werten öffnet sich die äußere Keratinfaserschicht optimal zur Aufnahme der Oxidationsfarbstoffvorprodukte und/oder entfaltet sich die oxidative Wirkung des Wasserstoffperoxids und ggf. weiterer Peroxidverbindungen optimal.

Weiterhin kann es erfindungsgemäß bevorzugt sein, das anwendungsbereite Färbe- oder Aufhellmittel durch Vermischen einer erfindungsgemäß verwendeten Aufhellzusammensetzung (A-1) mit einer erfindungsgemäß verwendeten Aufhellzusammensetzung (A-2) und einer erfindungsgemäß verwendeten Oxidationszusammensetzung (B) herzustellen.

Weiterhin kann es erfindungsgemäß bevorzugt sein, das anwendungsbereite Färbe- oder Aufhellmittel durch Vermischen einer erfindungsgemäß verwendeten Färbezusammensetzung (A-3) mit einer erfindungsgemäß verwendeten Aufhellzusammensetzung (A-2) und einer erfindungsgemäß verwendeten Oxidationszusammensetzung (B) herzustellen.

Erfindungsgemäß bevorzugte Verfahren zur, bevorzugt multitonalen, Farbveränderung keratinischer Fasern sind daher dadurch gekennzeichnet, dass das Gewichtsverhältnis von Färbe- oder Aufhellzusammensetzung (A) zu Oxidationszusammensetzung (B) im Bereich von 1 : 10 bis 4:1, bevorzugt im Bereich von 1 : 5 bis 3 : 1, besonders bevorzugt im Bereich von 1 : 4 bis 2 : 1 und außerordentlich bevorzugt im Bereich von 1:2 bis 1:1 liegt.

Um die Mischbarkeit von (A) und (B) und die Auswaschbarkeit des Färbemittels von den Keratinfasern nach Abschluss des erfindungsgemäßen Verfahrens zu begünstigen, ist es erfindungsgemäß bevorzugt, dass zumindest die Färbe- oder Aufhellzusammensetzung (A) mindestens ein Tensid enthält, bevorzugt in einer Gesamtmenge von 0,1 bis 15 Gew.-%, bevorzugt 1 bis 8 Gew.-%, bezogen auf das Gewicht der Zusammensetzung (A).

Auch die Zusammensetzung (B) enthält bevorzugt mindestens ein Tensid, bevorzugt in einer Gesamtmenge von 0,1 bis 10 Gew.-%, bevorzugt 1 bis 5 Gew.-%, bezogen auf das Gewicht der Zusammensetzung (B).

Geeignete Tenside, insbesondere für (A) und (B), sind nichtionische, anionische, kationische und amphotere Tenside. Bevorzugt sind nichtionische und anionische Tenside sowie Mischungen hiervon. Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül. Besonders bevorzugte amphotere Tenside sind Cocamidopropylbetain, N-Kokosalkylaminopropionat, Kokosacylaminoethylaminopropionat und $C_{12}$-$C_{18}$-Acylsarcosin.

Bevorzugte nichtionische Tenside sind ausgewählt aus ethoxylierten C10-C24-Fettalkoholen mit einem Ethoxylierungsgrad von 4 bis 120, bevorzugt 10 bis 50, besonders bevorzugt 12 bis 20, wobei Mischungen dieser ethoxylierten C10-C24-Fettalkohole mit unterschiedlichen Ethoxylierungsgraden besonders bevorzugt sind. Weitere bevorzugte nichtionische Tenside sind ausgewählt aus ethoxylierten Glycerinmono-, -di- und -triestern von C10-C24-Fettsäuren, beispielsweise PEG-40 Castor Oil, PEG-40 Hydrogenated Castor Oil, PEG-60 Castor Oil oder PEG-60 Hydrogenated Castor Oil. Weitere bevorzugte nichtionische Tenside sind ausgewählt aus $C_8$ - $C_{22}$-Alkylmono- und - oligoglycosiden. $C_8$ -$C_{22}$-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Handelsübliche Produkte, die zum Beispiel unter dem Warenzeichen Plantacare® erhältlich sind, enthalten eine glucosidisch gebundene $C_8$-$C_{16}$-Alkylgruppe an einem Oligoglucosidrest, dessen mittlerer Oligomerisationsgrad bei 1 - 2, insbesondere 1,2 - 1,4, liegt. Besonders bevorzugte $C_8$ - $C_{22}$-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon.

Bevorzugte kationische Tenside sind beispielsweise Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen.

Gelierzusammensetzung (C)

Um erfindungsgemäß die den Strähnchenfolien analog wirkenden Trennschichten aus Polysaccharidgel in situ auf den Keratinfasern herzustellen, wird auf die mit der Mischung (M) aus Färbe- oder Aufhellzusammensetzung (A) mit Oxidationszusammensetzung (B) behandelten Keratinfasern eine Gelierzusammensetzung (C) appliziert, beispielsweise mit einem Pinsel oder ähnlichen Applikator, oder - was besonders bevorzugt ist - durch Aufsprühen, wobei die Gelierzusammensetzung (C) enthält:

c)i. 0,1 - 3 Gew.-%, bevorzugt 0,2 - 2 Gew.-%, besonders bevorzugt 0,4 - 1 Gew.-%, bezogen auf das Gewicht der Zusammensetzung (C), mindestens eines Salzes eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, die Gelierzusammensetzung (C) 0,1 - 3 Gew.-%, bevorzugt 0,2 - 2 Gew.-%, besonders bevorzugt 0,4 - 1 Gew.-%,

bezogen auf das Gewicht der Zusammensetzung (C), mindestens eines Salzes, ausgewählt aus Kalium-, Calcium-, Strontium-, Barium- und Aluminiumsalzen mit einer Wasserlöslichkeit bei 20°C von mindestens 500 mg/l enthält,
c)ii. 50 - 99 Gew.-%, bezogen auf das Gewicht der Zusammensetzung (C), Wasser,
c)iii. optional mindestens ein Kationpolymer, das bevorzugt ausgewählt ist aus kationisierten Guar-Ethern;
c)iv. wobei die Gelierzusammensetzung (C) einen pH-Wert im Bereich von 4 bis 12, bevorzugt im Bereich von 4 - 9, besonders bevorzugt 6,5 - 8, aufweist, gemessen bei 20°C.

In Vorversuchen, die zu der vorliegenden Erfindung geführt haben, wurde festgestellt, dass das Einarbeiten eines Calciumsalzes mit einer Wasserlöslichkeit von mindestens 500 mg/l bei 20°C in eine erfindungsgemäße Färbezusammensetzung (A-3) die Entwicklung des Farbstoffs und die Anfärbung der Keratinfasern unter dem Einfluss des Wasserstoffperoxids deutlich beeinträchtigt hat. Für Aufhell- und Blondierverfahren wurde kein nachteiliger Effekt dieser Ausführungsform beobachtet.

Gel bildendes Salz / Wasserlöslichkeit

Das mindestens eine Salz c)i weist bei 20°C eine Wasserlöslichkeit von mindestens 500 mg/l, bevorzugt von mindestens 40 g/l, besonders bevorzugt von mindestens 200 g/l, außerordentlich bevorzugt von mindestens 350 g/l, auf. Das erfindungsgemäß außerordentlich bevorzugte Calciumchlorid hat eine Wasserlöslichkeit von 740 g/l bei 20°C. Andere erfindungsgemäß besonders bevorzugte Salze c)i sind ausgewählt aus Calciumacetat (Wasserlöslichkeit 374 g/l), Calciumlactat, Calciumgluconat, Calciumgluconatlactat. Ebenfalls bevorzugt sind Aluminiumchlorid, Aluminiumhydroxychlorid, Aluminiumsulfat, Aluminiumlactat, Aluminiumacetat, Aluminiumgluconat. Für die Gelierung von kappa-Carragheenan sind Kaliumchlorid, Kaliumacetat, Kaliumlactat, Kaliumgluconat und Kaliumsulfat bevorzugt, wobei Kaliumchlorid, Kaliumlactat, und Kaliumgluconat besonders bevorzugt sind. Geeignet sind weiterhin Strontiumchlorid und Bariumchlorid. Bevorzugt können auch Mischungen der vorgenannten Salze enthalten sein.

Besonders bevorzugt enthält die Gelierzusammensetzung (C) mindestens ein Salz, ausgewählt aus Calciumchlorid, Calciumchlorid, Calciumlactat, Calciumgluconat und Calciumgluconatlactat sowie Mischungen hiervon in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (C). Außerordentlich bevorzugt enthält die Gelierzusammensetzung (C) 0,1 - 5 Gew.-%, besonders bevorzugt 0,2 - 3 Gew.-%, außerordentlich bevorzugt 0,5 - 2,5 Gew.-%, Calciumchlorid, jeweils bezogen auf das Gewicht der Zusammensetzung (C).

Die Gelierzusammensetzung (C) enthält, jeweils bezogen auf ihr Gewicht, 50 - 99 Gew.-%, bevorzugt 70 - 95 Gew.-%, besonders bevorzugt 75 - 92 Gew.-% Wasser.

Die Gelierzusammensetzung (C) weist einen pH-Wert im Bereich von 4 bis 12, bevorzugt im Bereich von 4 - 9, besonders bevorzugt 6,5 - 8, auf, gemessen bei 20°C. Ob ein saurer oder ein alkalischer pH-Wert eingestellt ist, hängt unter anderem davon ab, ob die Viskosität der Zusammensetzung (C) zur Verbesserung ihrer Applikationseigenschaften, insbesondere zur Verbesserung des Sprühbildes, mit einem Verdickungsmittel angehoben werden soll. Zur bevorzugt moderaten Andickung der Zusammensetzung (C) sind besonders gut kationische Polymere geeignet. Bevorzugte kationische Polymere sind ausgewählt aus kationischen Guarderivaten, insbesondere aus kationisierten Guar-Ethern, insbesondere aus Polymeren mit den INCI-Bezeichnungen Hydroxypropyl Guar Hydroxypropyltrimonium Chloride und Guar Hydroxypropyltrimonium Chloride. Sofern die Gelierzusammensetzung (C) ein kationisches Polymer enthält, ist dieses in einer Gesamtmenge von 0,01 - 1 Gew.-%, bevorzugt 0,1 bis 0,8 Gew.-%, besonders bevorzugt 0,2 bis 0,5 Gew.-%, jeweils bezogen auf das Gewicht der Zusammensetzung (C), enthalten. Sofern die Gelierzusammensetzung (C) ein kationisches Polymer enthält, weist sie bevorzugt einen pH-Wert im Bereich von 4 - 6,5 auf, gemessen bei 20°C.

Die Einstellung des pH-Wertes erfolgt, wie auch für die übrigen erfindungsgemäß verwendeten Zusammensetzungen, mit üblichen pH-Stellmitteln, wie insbesondere Citronensäure, Milchsäure, NaOH, KOH und ähnlichen kosmetisch verträglichen Säuren und Basen.

In einer bevorzugten Ausführungsform des erfindungsgemäßen Farbveränderungsverfahrens wird die Gelierzusammensetzung (C) *in situ* oder erst 0,01 bis 24 Stunden vor Durchführung des erfindungsgemäßen Farbveränderungsverfahrens durch Vermischen einer festen, bevorzugt pulverförmigen, Gelierzusammensetzung (C'), enthaltend das mindestens eine unter c)i genannte Salz in Pulverform und optional mindestens ein Kationpolymer, das bevorzugt ausgewählt ist aus kationisierten Guar-Ethern, in Pulverform, mit Wasser c) iii hergestellt. Besonders bevorzugt enthält die Gelierzusammensetzung (C') zusätzlich ein festes, bevorzugt pulverförmiges, pH-Stellmittel, z. B. eine Säure wie Citronensäure, und/oder ein Alkalisierungsmittel, wie Natriumsilikat.

Polysaccharid-Zusammensetzung (D)

Eine besonders bevorzugte Ausführungsform des erfindungsgemäßen Farbveränderungsverfahrens ist dadurch gekennzeichnet, dass das mindestens eine Polysaccharid, das mit Calciumionen in wässrigem Medium ein Gel

bilden kann und das bevorzugt ausgewählt ist aus Alginsäure, kappa-Carragheenan, iota-Carragheenan und Pektin, und/ oder mindestens einem Salz der vorgenannten Polysaccharide, das ausgewählt ist aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist, bereitgestellt wird in Form einer Polysaccharid-Zusammensetzung (D), enthaltend

d)i. mindestens ein Polysaccharid, das mit Calciumionen in wässrigem Medium ein Gel bilden kann und bevorzugt ausgewählt ist aus Alginsäure, kappa-Carragheenan, iota-Carragheenan und Pektin, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, bevorzugt in einer Gesamtmenge von 0,1 - 5 Gew.-%, bevorzugt 0,5 - 2 Gew.-%, besonders bevorzugt 0,5 - 2,5 Gew.-%, jeweils bezogen auf das Gewicht der Zusammensetzung (D), wobei im Falle von Salzen des kappa-Carragheenans dessen Alkalimetall-Salz nur aus Lithium- und Natriumsalzen ausgewählt ist,
d)ii. 50 - 99,9, bevorzugt 60 bis 95, besonders bevorzugt 80 bis 90 Gew.-% Wasser, jeweils bezogen auf das Gewicht der Zusammensetzung (D),
d)iii. optional mindestens ein Alkalisierungsmittel, das bevorzugt ausgewählt ist aus pulverförmigen Natriumsilikaten, insbesondere pulverförmigen Natriummetasilikaten,
d)iv. optional mindestens ein Polymer, enthaltend (Meth)Acrylsäure-, (Meth)Acrylsäureester-, (Meth)Acrylamid- und/oder 2-Acrylamido-2-methylpropansulfonsäure-Monomere, bevorzugt in einer Gesamtmenge von 0,05 - 3 Gew.-%, bevorzugt 0,2 - 0,5 Gew.-%, bezogen auf das Gewicht der Zusammensetzung (D),
d)v. optional mindestens ein Öl in einer Gesamtmenge von 0,1 bis 10 Gew.-%, bevorzugt 0,3 bis 5 Gew.-%, besonders bevorzugt 1 bis 3 Gew.-%, jeweils bezogen auf das Gewicht der Polysaccharid-Zusammensetzung (D),

wobei optional die Polysaccharid-Zusammensetzung (D) durch Vermischen einer festen, bevorzugt pulverförmigen, Polysaccharid-Zusammensetzung (D'), enthaltend d)i und optional d)iii und/oder optional d)iv, mit der Komponente d)ii und optional mit der Komponente d)v *in situ* oder 0,01 bis 24 Stunden vor Durchführung des erfindungsgemäßen Farbveränderungsverfahrens hergestellt wird,

In einer bevorzugten Ausführungsform des erfindungsgemäßen Farbveränderungsverfahrens wird die Polysaccharid-Zusammensetzung (D) *in situ* oder 0,01 bis 24 Stunden vor Durchführung des erfindungsgemäßen Farbveränderungsverfahrens durch Vermischen einer festen, bevorzugt pulverförmigen, Polysaccharid-Zusammensetzung (D'), enthaltend d)i und optional d)iii und/oder optional d)iv, mit der Komponente d)ii und optional mit der Komponente d)v hergestellt.

Bei der in situ Herstellung der Polysaccharid-Zusammensetzung (D) hat es sich bewährt, das mindestens eine Polysaccharid, das mit Calciumionen in wässrigem Medium ein Gel bilden kann, in 25°C bis 40 °C warmes Wasser aufzulösen, das mindestens ein Öl in einer Gesamtmenge von 0,1 bis 10 Gew.-%, bevorzugt 0,3 bis 5 Gew.-%, besonders bevorzugt 1 bis 3 Gew.-%, jeweils bezogen auf das Gewicht der Polysaccharid-Zusammensetzung (D), enthält. Das Öl sollte vor der Zugabe des Polysaccharids im Wasser enthalten sein. Dadurch wird die Auflösung des Polysaccharids deutlich begünstigt. Erfindungsgemäß außerordentlich bevorzugte Öle sind ausgewählt aus natürlichen und synthetischen Kohlenwasserstoffen, besonders bevorzugt aus Mineralölen, Paraffinölen, $C_{18}$-$C_{30}$-Isoparaffinen, insbesondere Isoeicosan, Polyisobutene und Polydecene, weiterhin ausgewählt aus $C_8$-$C_{16}$-Isoparaffinen, insbesondere aus Isodecan, Isododecan, Isotetradecan und Isohexadecan sowie Mischungen hiervon, sowie 1,3-Di-(2-ethylhexyl)-cyclohexan.

Weitere erfindungsgemäß bevorzugte Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Cetyl-2-ethylhexanoat, 2-Hexyldecylstearat (z. B. Eutanol® G 16 S), 2-Hexyldecyllaurat, Isodecylneopentanoat, Isononylisononanoat, 2-Ethylhexylpalmitat (z. B. Cegesoft® C 24) und 2-Ethylhexylstearat (z. B. Cetiol® 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß bevorzugte Öle sind ausgewählt aus den Benzoesäureestern von linearen oder verzweigten C8-22-Alkanolen. Besonders bevorzugt sind Benzoesäure-C12-C15-alkylester, z. B. erhältlich als Handelsprodukt Finsolv® TN, Benzoesäureisostearylester, z. B. erhältlich als Handelsprodukt Finsolv® SB, Ethylhexylbenzoat, z. B. erhältlich als Handelsprodukt Finsolv® EB, und Benzoesäureoctyldocecylester, z. B. erhältlich als Handelsprodukt Finsolv® BOD.

Weitere erfindungsgemäß bevorzugte Öle sind ausgewählt aus Fettalkoholen mit 6 - 30 Kohlenstoffatomen, die ungesättigt oder verzweigt und gesättigt oder verzweigt und ungesättigt sind. Die verzweigten Alkohole werden

häufig auch als Guerbet-Alkohole bezeichnet, da sie nach der Guerbet-Reaktion erhältlich sind. Bevorzugte Alkoholöle sind 2-Hexyldecanol (Eutanol® G 16), 2-Octyldodecanol (Eutanol® G), 2-Ethylhexylalkohol und Isostearylalkohol.

Weitere bevorzugte Öle sind ausgewählt aus Mischungen aus Guerbetalkoholen und Guerbetalkoholestern, z.B. dem Handelsprodukt Cetiol® PGL (2-Hexyldecanol und 2-Hexyldecyllaurat).

Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/ Caprylic Triglycerides, z. B. die Handelsprodukte Myritol® 318, Myritol® 331 (BASF) oder Miglyol® 812 (Hüls) mit unverzweigten Fettsäureresten sowie Glyceryltriisostearin mit verzweigten Fettsäureresten.

Weitere erfindungsgemäß besonders bevorzugte kosmetische Öle sind ausgewählt aus den Dicarbonsäureestern von linearen oder verzweigten C2-C10-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexyl-succinat und Di-(2-hexyldecyl)-succinat.

Weitere erfindungsgemäß bevorzugte kosmetische Öle sind ausgewählt aus den Anlagerungsprodukten von 1 bis 5 Propylenoxid-Einheiten an ein- oder mehrwertige C8-22-Alkanole, wie Octanol, Decanol, Decandiol, Laurylalkohol, Myristylalkohol und Stearylalkohol, z. B. PPG-2-Myristylether und PPG-3-Myristylether (Witconol® APM).

Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C3-22-Alkanole wie Glycerin, Butanol, Butandiol, Myristylalkohol und Stearylalkohol, die gewünschtenfalls verestert sein können, z. B. PPG-14-Butylether (Ucon Fluid® AP), PPG-9-Butylether (Breox® B25), PPG-10-Butandiol (Macol® 57), PPG-15-Stearylether (Arlamol® E) und Glycereth-7-diisononanoat.

Weitere erfindungsgemäß bevorzugte kosmetische Öle sind ausgewählt aus den C8-C22-Fettalkoholestern einwertiger oder mehrwertiger C2-C7-Hydroxycarbonsäuren, insbesondere die Ester der Glycolsäure, Milchsäure, Äpfelsäure, Weinsäure, Citronensäure und Salicylsäure. Solche Ester auf Basis von linearen C14/15-Alkanolen, z. B. C12-C15-Alkyllactat, und von in 2-Position verzweigten C12/13-Alkanolen sind unter dem Warenzeichen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI.

Weitere erfindungsgemäß bevorzugte kosmetische Öle sind ausgewählt aus den symmetrischen, unsymmetrischen oder cyclischen Estern der Kohlensäure mit $C_{3-22}$-Alkanolen, $C_{3-22}$-Alkandiolen oder $C_{3-22}$-Alkantriolen, z. B. Dicaprylylcarbonat (Cetiol® CC) oder die Ester gemäß der Lehre der DE19756454 A1, insbesondere Glycerincarbonat.

Weitere kosmetische Öle, die erfindungsgemäß bevorzugt sein können, sind ausgewählt aus den Estern von Dimeren ungesättigter $C_{12}$-$C_{22}$-Fettsäuren (Dimerfettsäuren) mit einwertigen linearen, verzweigten oder cyclischen $C_2$-$C_{18}$-Atkanoten oder mit mehrwertigen linearen oder verzweigten $C_2$-$C_6$-Alkanolen.

Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Bevorzugt können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan ($L_2$), Octamethyltrisiloxan ($L_3$), Decamethyltetrasiloxan ($L_4$) sowie beliebige Zweier- und Dreiermischungen aus $L_2$, $L_3$ und/ oder $L_4$, bevorzugt solche Mischungen, wie sie z. B. in den Handelsprodukten DC 2-1184, Dow Corning® 200 (0,65 cSt) und Dow Corning® 200 (1,5 cSt) von Dow Corning enthalten sind. Bevorzugte nichtflüchtige Siliconöle sind ausgewählt aus höhermolekularen linearen Dimethylpolysiloxanen, im Handel erhältlich z. B. unter der Bezeichnung Dow Corning® 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von etwa 350 cSt. Es kann erfindungsgemäß außerordentlich bevorzugt sein, Mischungen der vorgenannten Öle einzusetzen.

Auch durch Zugabe eines Tensids, zum Beispiel in einer Gesamtmenge von 0,05 bis 3 Gew.-%, bevorzugt 0,1 bis 1 Gew.-%, besonders bevorzugt 0,3 bis 0,8 Gew.-%, bezogen auf das Gewicht der Zusammensetzung (D), kann die Auflösung des Polysaccharids günstig beeinflusst werden. Allerdings kann ein Tensidgehalt bewirken, dass die Polysaccharid-Zusammensetzung (D), die zum Lösen des Polysaccharids geschüttelt werden sollte, zu stark schäumt, was ihre Applikation erschwert. Bevorzugt enthält die Polysaccharid-Zusammensetzung (D) daher kein Tensid.

Besonders bevorzugt verwendete Polysaccharid-Zusammensetzungen (D) und (D') sind dadurch gekennzeichnet, dass das mindestens eine Polysaccharid, das mit Calciumionen in wässrigem Medium ein Gel bilden kann, ausgewählt ist aus Alginsäure, Natriumalginat, Ammoniumalginat, Magnesiumalginat, Monoethanolammoniumalginat, kappa-Carragheenan, das Lithiumsalz von kappa-Carragheenan, das Natriumsalz von kappa-Carragheenan, iota-Carragheenan, das Lithiumsalz von iota-Carragheenan, das Natriumsalz von iota-Carragheenan, das Kaliumsalz von iota-Carragheenan, das Ammoniumsalz von iota-Carragheenan, Pektin, Natriumpektinat, Ammoniumpektinat, Kaliumpektinat und Magnesiumpektinat, sowie Mischungen dieser Substanzen. Erfindungsgemäß außerordentlich bevorzugt sind Mischungen aus Alginsäure und Natriumalginat, insbesondere Mischungen aus Alginsäure und Natriumalginat im Gewichtsverhältnis von Alginsäure zu Natriumalginat im Bereich von 1:2 bis 2:1, bevorzugt im Bereich von 0,8 bis 1,25, besonders bevorzugt im Gewichtsverhältnis 1:1.

Besonders bevorzugt enthält die Polysaccharid-Zusammensetzung (D) eine Mischung aus Alginsäure und Natriumalginat in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (D), wobei es bevorzugt ist, wenn das Gewichtsverhältnis von Alginsäure zu Natriumalginat im Bereich von 1:2 bis 2:1, bevorzugt im Bereich von 0,8 bis 1,25, besonders bevorzugt im Gewichtsverhältnis 1:1 liegt.

Es ist erfindungsgemäß bevorzugt, dass die Polysaccharid-Zusammensetzung (D) einen pH-Wert im Bereich von 7 bis 12, bevorzugt 8 bis 10, besonders bevorzugt 8,5 bis 9,5, aufweist, jeweils bei 20°C gemessen. Daher enthält die Polysaccharid-Zusammensetzung (D) bevorzugt mindestens ein Alkalisierungsmittel, das besonders bevorzugt ausgewählt ist aus pulverförmigen Natriumsilikaten, insbesondere pulverförmigen Natriummetasilikaten. Aber auch die anderen vorstehend aufgeführten Alkalisierungsmittel können zur Einstellung des pH-Werts der Polysaccharid-Zusammensetzung (D) eingesetzt werden. Für die Polysaccharid-Zusammensetzung (D') ist ein Gehalt an mindestens einem festen, bevorzugt pulverförmigen, Alkalisierungsmittel bevorzugt, das bevorzugt ausgewählt ist aus pulverförmigen Natriumsilikaten, insbesondere pulverförmigen Natriummetasilikaten.

Es kann erfindungsgemäß bevorzugt sein, dass die Polysaccharid-Zusammensetzung (D) oder die Polysaccharid-Zusammensetzung (D') mindestens eine Polymer, enthaltend (Meth)Acrylsäure-, (Meth)Acrylsäureester-, (Meth)Acrylamid- und/oder 2-Acrylamido-2-methylpropansulfonsäure-Monomere, enthält, bevorzugt in einer Gesamtmenge von 0,05 - 3 Gew.-%, bevorzugt 0,2 - 0,5 Gew.-%, bezogen auf das Gewicht der Zusammensetzung (D). Der Zusatz dieser Polymere dient vor allem dazu, die Löslichkeit des Polysaccharids bei der in situ-Herstellung der Zusammensetzung (D) zu verbessern. Bevorzugt ist das mindestens eine Polymer, enthaltend (Meth)Acrylsäure-, (Meth)Acrylsäureester-, (Meth)Acrylamid- und/oder 2-Acrylamido-2-methylpropansulfonsäure-Monomere, ausgewählt aus vernetzten Homopolymeren der Acrylsäure, vernetzten Homopolymeren der 2-Acrylamido-2-methylpropansulfonsäure, vernetzten Copolymeren bestehend aus Acrylsäure- und 2-Acrylamido-2-methylpropansulfonsäure-Einheiten, vernetzten Copolymeren bestehend aus 2-Methyl-2[(1-oxo-2-propenyl)amino]-1-propansulfonsäure-Einheiten und Hydroxyethylacrylat-Einheiten, vernetzten Copolymeren bestehend aus Acrylamid- und 2-Acrylamido-2-methylpropansulfonsäure-Einheiten, vernetzten Copolymeren bestehend aus Methacrylsäure-Einheiten und C1-C4-Alkylacrylat-Einheiten, vernetzten Copolymeren bestehend aus Acrylsäure-Einheiten und C1-C4-Alkylmethacrylat-Einheiten, sowie Mischungen hiervon.

Besonders bevorzugt weist das mindestens eine Polymer, enthaltend (Meth)Acrylsäure-, (Meth)Acrylsäureester-, (Meth)Acrylamid- und/oder 2-Acrylamido-2-methylpropansulfonsäure-Monomere, keine Monomere auf, die mit ggf. ethoxylierten, $C_6$ - $C_{24}$-Alkyl- oder $C_6$ - $C_{24}$-AlkenylGruppen substituiert sind.

Für den Einsatz der Polysaccharid-Zusammensetzung (D) gibt es drei erfindungsgemäß bevorzugte Ausführungsformen des erfindungsgemäßen Farbänderungsverfahrens.

Ein erstes erfindungsgemäß bevorzugtes Farbänderungsverfahrens ist dadurch gekennzeichnet, dass die Polysaccharid-Zusammensetzung (D) in einem Verfahrensschritt nach dem Applikationsschritt e und vor dem Applizieren, bevorzugt Aufsprühen, der Gelierzusammensetzung (C) auf die mit der Mischung (M) aus (A) und (B) beaufschlagte(n) keratinische(n) Faser(n) appliziert, bevorzugt aufgesprüht, wird.

Dieses Verfahren ist am meisten bevorzugt, denn hierbei tritt die beste Trennkraft auf, das heißt, zwischen benachbarten Keratinfasersträhnen mit unterschiedlicher Färbung tritt trotz des gegenseitigen Kontakts während der Einwirkzeit kein Abfärben oder Verschmieren der Farbe auf. Auch tritt bei diesem Verfahren keine Farbverschiebung auf.

Ein zweites erfindungsgemäß bevorzugtes Farbänderungsverfahrens ist dadurch gekennzeichnet, dass die Polysaccharid-Zusammensetzung (D) in einem Verfahrensschritt vor dem Applikationsschritt e zu einer der Zusammensetzungen (A) oder (B) oder zur Mischung (M) aus (A) und (B) gegeben wird.

Auch bei diesem Verfahren tritt keine für das ungeschulte Auge sichtbare Farbverschiebung auf. Die Trennkraft ist ebenfalls sehr gut, allerdings etwas schlechter als bei dem vorstehend genannten Verfahren, bei dem (D) nicht in (A) oder (B) oder (M) enthalten ist, sondern auf die mit /(M) beaufschlagten Keratinfasern appliziert, bevorzugt gesprüht, wird.

Ein drittes erfindungsgemäß bevorzugtes Farbänderungsverfahrens ist dadurch gekennzeichnet, dass die Polysaccharid-Zusammensetzung (D) in einem Verfahrensschritt nach dem Applizieren, bevorzugt Aufsprühen, der Gelierzusammensetzung (C) auf die mit der Mischung (M) aus (A) und (B) beaufschlagte(n) keratinische(n) Faser(n) appliziert, bevorzugt aufgesprüht, wird.

Auch tritt bei diesem Verfahren keine Farbverschiebung auf. Die Trennkraft ist geringfügig schlechter als bei dem vorstehend genannten ersten Verfahren, bei dem erst (D), dann (C) appliziert wird, aber immer noch besser als bei dem zweiten Verfahren mit dem Polysaccharid in (A) oder (B) oder (M).

Für die drei vorgenannten bevorzugten Ausführungsformen des erfindungsgemäßen Farbänderungsverfahrens ist es besonders bevorzugt, dass das Gewichtsverhältnis von Polysaccharid-Zusammensetzung (D) zur Mischung (M) aus Färbe- oder Aufhellzusammensetzung (A) und Oxidationszusammensetzung (B) im Bereich von 1 : 20 bis 1:2, bevorzugt im Bereich von 1:10 bis 1:5 liegt.

Für die drei vorgenannten bevorzugten Ausführungsformen des erfindungsgemäßen Farbänderungsverfahrens ist es weiterhin besonders bevorzugt, dass das Gewichtsverhältnis von Polysaccharid-Zusammensetzung (D) zur Gelierzusammensetzung (C) im Bereich von 0,2 : 1 bis 2:1, bevorzugt im Bereich von 0,5 : 1 bis 1,5 : 1 liegt und besonders bevorzugt 1:1 beträgt.

Für alle erfindungsgemäßen und erfindungsgemäß bevorzugten Ausführungsformen des vorliegenden Farbänderungsverfahrens ist es weiterhin besonders bevorzugt, dass das Gewichtsverhältnis von Gelierzusammensetzung (C) zur Mischung (M) aus Färbe- oder Aufhellzusammensetzung (A) und Oxidationszusammensetzung (B) im Bereich von 1 : 20 bis 1:2, bevorzugt im Bereich von 1:10 bis 1:5 liegt.

Für alle erfindungsgemäßen und erfindungsgemäß bevorzugten Ausführungsformen des vorliegenden Farbänderungsverfahrens ist es weiterhin besonders bevorzugt, dass es mit mindestens einer zweiten Färbe- oder Aufhellzusammensetzung (A'), (A"), (A'") usw. anstelle von (A) durchgeführt wird, wobei sich die Zusammensetzungen (A) und (A'), (A"), (A'") usw. zumindest in Bezug auf die enthaltenen Oxidationsfarbstoffvorprodukte und/oder direktziehenden Haarfarbstoffe qualitativ und/oder quantitativ voneinander unterscheiden.

Für alle erfindungsgemäßen und erfindungsgemäß bevorzugten Ausführungsformen des vorliegenden Farbänderungsverfahrens ist es weiterhin besonders bevorzugt, dass die Keratinfasersträhnen nicht mit festen flächigen Trennhilfen, insbesondere nicht mit Folien aus festen Materialien, wie Aluminium, Papier oder Styropor, versehen sind.

Ein weiterer Gegenstand der vorliegenden Erfindung ist ein Kit zur, bevorzugt multitonalen, Farbveränderung keratinischer Fasern, umfassend die folgenden, physikalisch voneinander getrennten Komponenten (D) und (C) sowie optional (A) und optional (B):

I. eine Polysaccharid-Zusammensetzung (D), enthaltend

d)i. mindestens ein Polysaccharid, das mit Calciumionen in wässrigem Medium ein Gel bilden kann und bevorzugt ausgewählt ist aus Alginsäure, kappa-Carragheenan, iota-Carragheenan und Pektin, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist, wobei Mischungen aus Alginsäure und Natriumalginat besonders bevorzugt sind,

d)ii. mindestens ein Alkalisierungsmittel, das bevorzugt ausgewählt ist aus pulverförmigen Natriumsilikaten, insbesondere pulverförmigen Natriummetasilikaten,

d)iii. optional mindestens ein Polymer, enthaltend (Meth)Acrylsäure-, (Meth)Acrylsäureester-, (Meth)Acrylamid- und/oder 2-Acrylamido-2-methylpropansulfonsäure-Monomere, das bevorzugt keine Monomere aufweist, die mit ggf. ethoxylierten, $C_6$ - $C_{24}$-Alkyl- oder $C_6$ - $C_{24}$-Alkenyl-Gruppen substituiert sind;

II. eine Gelierzusammensetzung (C'), enthaltend

c)i. mindestens ein Salz eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, mindestens ein Salz, ausgewählt aus Kalium-, Calcium-, Strontium-, Barium- und Aluminiumsalzen mit einer Wasserlöslichkeit bei 20°C von mindestens 500 mg/l;

c)ii. optional mindestens ein Kationpolymer, das bevorzugt ausgewählt ist aus kationisierten Guar-Ethern,

III. optional eine Färbe- oder Aufhellzusammensetzung (A), enthaltend

a)i mindestens ein Alkalisierungsmittel,

a)ii optional mindestens einen Konsistenzgeber und

a)iii optional mindestens ein Lösemittel, ausgewählt aus Wasser, einem oder mehreren ein- oder mehrwertigen Alkoholen mit 2 bis 9 Kohlenstoffatomen sowie Mischungen dieser Lösemittel und

a)iv optional mindestens ein Oxidationsfarbstoffvorprodukt und/oder mindestens einen direktziehenden Haarfarbstoff,

wobei die Färbe- oder Aufhellzusammensetzung (A), sofern sie, bezogen auf ihr Gewicht, mehr als 7 Gew.-% Wasser enthält, einen pH-Wert im Bereich von 6,0 bis 11,5 aufweist, gemessen bei 20°C, und sofern die Zusammensetzung (A), bezogen auf ihr Gewicht, 0 bis 7 Gew.-% Wasser enthält, ihre 30 Gew.-%ige Dispersion in Wasser einen pH-Wert im Bereich von 6,0 bis 12,0, bevorzugt 7,5 - 11,5, besonders bevorzugt 8 bis 11,0, aufweist, jeweils gemessen bei 20°C;
IV. optional eine Oxidationszusammensetzung (B), enthaltend

b)i. 1 - 18 Gew.-% Wasserstoffperoxid, bezogen auf das Gewicht der Zusammensetzung (B), und
b)ii. Wasser,
b)iii. wobei die Oxidationszusammensetzung (B) einen pH-Wert im Bereich von einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen.

Für erfindungsgemäße und erfindungsgemäß bevorzugte Kits gilt mutatis mutandis das vorstehend zu den erfindungsgemäßen und erfindungsgemäß bevorzugten Farbveränderungsverfahren. Erfindungsgemäße und erfindungsgemäß bevorzugte Kits haben den Vorteil, dass die wesentlichen Inhaltsstoffe der Zusammensetzungen (C) und (D) in platz- und verpackungssparender Weise, beispielsweise in Sachets, die bevorzugt eine wasserdampfundurchlässige Schicht aufweisen, kommerzialisiert werden können. So kann beispielsweise der Friseur durch einfaches Vermischen mit der Zusammensetzungen (C') und (D') mit Wasser und ggf. einer öligen Haarpflegezusammensetzung die Zusammensetzungen (C) und (D) selbst herstellen. Bevorzugt umfasst das Kit auch eine Gebrauchsanweisung für die Durchführung erfindungsgemäßer und erfindungsgemäß bevorzugter Farbveränderungsverfahren.

Ein weiterer Gegenstand der vorliegenden Erfindung ist die Verwendung von mindestens einem Polysaccharidsalz, ausgewählt aus Calciumalginat, Kalium-kappa-Carragheenat, Calcium-kappa-Carragheenat, Calcium-iota-Carragheenat, Calciumpektinat, Aluminiumalginat, Aluminium-kappa-Carragheenat, Aluminium-iota-Carragheenat, Aluminiumpektinat, Strontiumalginat, Strontium-kappa-Carragheenat, Strontium-iota-Carragheenat, Strontiumpektinat, Bariumalginat, Bariumkappa-Carragheenat, Barium-iota-Carragheenat, Bariumpektinat, sowie Mischungen dieser Polysaccharidsalze, zur Beschichtung von Keratinfaserpartien oder Keratinfasersträhnen in einem Verfahren zur, bevorzugt multitonalen, Farbveränderung keratinischer Fasern, wobei das Farbveränderungsverfahren bevorzugt ein Verfahren gemäß einem der Ansprüche 1 - 10, 12 oder 13 ist.

Für diese erfindungsgemäße Verwendung und bevorzugte Ausführungsformen gilt mutatis mutandis das vorstehend zu den erfindungsgemäßen und erfindungsgemäß bevorzugten Farbveränderungsverfahren und Kits.

Versuchsergebnisse

Tabelle 1: Aufhell- oder Färbecreme (A-1), alle Mengenangaben in Gew.-%

| | Färbecreme 1.1 | Färbecreme 1.2 | Aufhellcreme 1.3 | Aufhellcreme 1.4 |
|---|---|---|---|---|
| Cetearyl Alcohol | 15,0 | 15,0 | 15,0 | 15,0 |
| Ammonium Hydroxide | 6,3 | 6,3 | 6,3 | 6,3 |
| Glyceryl Stearate | 3,5 | 3,5 | 3,5 | 3,5 |
| Ceteareth-20 | 3,0 | 3,0 | 3,0 | 3,0 |
| Octyldodecanol | 4 | 4 | 4 | 4 |
| Sodium Laureth Sulfate | 1,2 | 1,2 | 1,2 | 1,2 |
| Glycerin | 1,5 | 1,5 | 1,5 | 1,5 |
| Sodium Cetearyl Sulfate | 0,7 | 0,7 | 0,7 | 0,7 |
| Oleic Acid | 0,4 | 0,4 | 0,4 | 0,4 |
| Parfum (Fraqrance) | 0,3 | 0,3 | 0,3 | 0,3 |
| Potassium Stearate | 0,3 | 0,3 | 0,3 | 0,3 |
| Tetrasodium EDTA | 0,17 | 0,17 | 0,17 | 0,17 |

(fortgesetzt)

| | Färbecreme 1.1 | Färbecreme 1.2 | Aufhellcreme 1.3 | Aufhellcreme 1.4 |
|---|---|---|---|---|
| Carbomer | 0,2 | 0,2 | 0,2 | 0,2 |
| Polyquaternium-39 | 0,1 | 0,1 | 0,1 | 0,1 |
| Sodium Sulfite | 0,1 | 0,1 | 0,1 | 0,1 |
| Potassium Hydroxide | 0,08 | 0,08 | 0,08 | 0,08 |
| Ascorbic Acid | 0,05 | 0,05 | 0,05 | 0,05 |
| Linoleamidopropyl PG-Dimonium Chloride Phosphate | 0,03 | 0,03 | 0,03 | 0,03 |
| 1-Hydroxyethyl 4,5-Diamino Pyrazole Sulfate | 1,5 | 1,5 | - | - |
| 4-Amino-3-methylphenol | 0,18 | 0,18 | - | - |
| 4-Amino-2-Hydroxytoluene | 0,3 | 0,3 | - | - |
| m-Aminophenol | 0,6 | 0,6 | - | - |
| Citric Acid | 0,002 | 0,002 | 0,002 | 0,002 |
| Alginsäure | - | 1,25 | - | 1,25 |
| Natriumalginat | - | 1,25 | - | 1,25 |
| Aqua (Water, Eau) | ad 100 | ad 100 | ad 100 | ad 100 |

Tabelle 2: Oxidationszusammensetzung (B), alle Mengenangaben in Gew.-%

| | |
|---|---|
| Paraffinum liquidum | 17,0 |
| Hydrogen Peroxide | 6,0 |
| Cetearyl Alcohol | 4,0 |
| PEG-40 Castor Oil | 0,8 |
| Sodium Cetearyl Sulfate | 0,5 |
| Etidronic Acid | 0,2 |
| Potassium Hydroxide | 0,12 |
| Disodium Pyrophosphate | 0,1 |
| 2,6-Dicarboxypyridine | 0,1 |
| Sodium Benzoate | 0,01 |
| Aqua (Water, Eau) | ad 100 |

Filmbildendes Polysaccharid wird aufgesprüht

Färbecreme 1.1 oder Aufhellcreme 1.3 gemäß Tabelle 1 wurden jeweils im Gewichtsverhältnis 1:1 mit der: Oxidationszusammensetzung (B) gemäß Tabelle 2 vermischt.

Das Haar einer Humanhaarperücke wurde halbseitig strähnchenweise eingefärbt, abwechselnd mit Färbecreme und mit Blondiercreme. Nachdem jeweils eine Strähne mit der 1:1-Mischung aus Färbecreme 1.1 + B beaufschlagt worden war, wurde eine wässrige Lösung (D), enthaltend 1,25 Gew.-% Alginsäure, 1,25 Gew.-% Natriumalginat, Natriummetasilikat bis pH 8,5 und 1 Gew.-% C10-C13-Isoparaffin sowie Wasser ad 100 Gew.-% aufgesprüht.

Anschließend wurde eine wässrige Lösung (C), enthaltend 1 Gew.-% Calciumchlorid, 0,5 Gew.-% Hydroxypropylguarhydroxypropyltrimoniumchlorid, Citronensäure bis pH 4 und Wasser ad 100 Gew.-% aufgesprüht. Die Strähne wurde spürbar fester.

Anschließend wurde eine benachbarte Strähne mit der 1:1-Mischung aus Aufhellcreme 1.3 + B beaufschlagt,

mit der vorgenannten wässrigen Lösung (D) und anschließend mit der wässrigen Lösung (C) besprüht. Die Strähne verfestigte sich spürbar.

Bei allen Sprühvorgängen wurde die Kopfhälfte, die mit der Mischung aus 1.2+B und 1.4+B behandelt wurde, mit einem Handtuch abgedeckt, um den Vergleichstest nicht zu verfälschen.

Die andere Halbseite der Perücke wurde entsprechend strähnchenweise eingefärbt, abwechselnd mit Färbecreme 1.2+B und mit Blondiercreme 1.4+B. Die Strähnen wurden jeweils nur mit der vorgenannten wässrigen Lösung (C) besprüht. Die Strähnen verfestigten sich dabei spürbar.

Nachdem das gesamte Haupthaar eingefärbt worden war, wurden die Strähnen vorsichtig mit der Hand an den Kopf angedrückt. Nach 45 Minuten Einwirkzeit bei 20°C Umgebungstemperatur ohne weitere Wärmezufuhr wurden die Haare mit Wasser und einem Shampoo ausgewaschen, mit einem Conditioner behandelt und erneut ausgewaschen, dann mit dem Handtuch getrocknet und trockengefönt.

Die farbmetrische Auswertung zeigte keine Farbverschiebung zu den Ausfärbungen ohne Polysaccharid-Calcium-Gel.

Für die mit D und C besprühten Strähnen mit 1.1 und 1.3 ergab sich eine bessere Trennkraft, das heißt eine geringere Färbe-/Aufhellmittel-Verunreinigung zwischen benachbarten unterschiedlich ange- bzw. entfärbten Strähnen als für das Verfahren mit den Zusammensetzungen 1.2 und 1.4.

## Patentansprüche

**1.** Verfahren zur Farbveränderung keratinischer Fasern, **gekennzeichnet durch** folgende Verfahrensschritte:

a. Bereitstellen einer Färbe- oder Aufhellzusammensetzung (A), enthaltend

a)i. mindestens ein Alkalisierungsmittel,
a)v. wobei die Färbe- oder Aufhellzusammensetzung (A), sofern sie, bezogen auf ihr Gewicht, mehr als 7 Gew.-% Wasser enthält, einen pH-Wert im Bereich von 6,0 bis 11,5 aufweist, gemessen bei 20°C, und sofern die Zusammensetzung (A), bezogen auf ihr Gewicht, 0 bis 7 Gew.-% Wasser enthält, sie so zusammengesetzt ist, dass ihre 30 Gew.-%ige Dispersion in Wasser einen pH-Wert im Bereich von 6,0 bis 12,0 aufweist, jeweils gemessen bei 20°C;

b. Bereitstellen einer Oxidationszusammensetzung (B), enthaltend

b)i. 1 - 18 Gew.-% Wasserstoffperoxid, bezogen auf das Gewicht der Zusammensetzung (B), und
b)ii. Wasser,
b)iii. wobei die Oxidationszusammensetzung (B) einen pH-Wert im Bereich von einen pH-Wert im Bereich von 2,5 - 6,5 aufweist, jeweils bei 20°C gemessen;

c. Bereitstellen einer Gelierzusammensetzung (C), enthaltend

c)i. 0,1 - 3 Gew.-%, bezogen auf das Gewicht der Zusammensetzung (C), mindestens eines Salzes eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, die Gelierzusammensetzung (C) 0,1 - 3 Gew.-%, bezogen auf das Gewicht der Zusammensetzung (C), mindestens eines Salzes, ausgewählt aus Kalium-, Calcium-, Strontium-, Barium- und Aluminiumsalzen mit einer Wasserlöslichkeit bei 20°C von mindestens 500 mg/l enthält,
c)ii. 50 - 99 Gew.-%, bezogen auf das Gewicht der Zusammensetzung (C), Wasser,
c)iv. wobei die Gelierzusammensetzung (C) einen pH-Wert im Bereich von 4 bis 12, aufweist, gemessen bei 20°C;

anschließend, das heißt, 1 bis 600 Sekunden danach,
d. Herstellen einer Mischung (M) aus (A) und (B), die einen pH-Wert im Bereich von 6,0 bis 11 aufweist, jeweils gemessen bei 20°C; nach 1 bis 600 Sekunden dann
e. Applikation zumindest einer Teilmenge von (M) auf mindestens eine zu färbende und/oder aufzuhellende Keratinfaserpartie;
1 bis 600 Sekunden danach
f. Applizieren der Gelierzusammensetzung (C) auf die mit (M) beaufschlagte(n) Keratinfaserpartie(n);

g. Wiederholen der Schritte e. und f. im gewünschten Umfang,

h. für eine Zeit von 0,1 bis 60 Minuten Belassen auf den keratinischen Fasern, anschließend Spülen der keratinischen Fasern mit Wasser und ggf. einem Reinigungsmittel, ggf. Nachbehandlung der Fasern mit einem Konditioniermittel und anschließendes Trocknen, **dadurch gekennzeichnet, dass** das Verfahren die Applikation von mindestens einem Polysaccharid, das mit Calciumionen in wässrigem Medium ein Gel bilden kann, umfasst, wobei diese Polysaccharide nicht in der Gelierzusammensetzung (C) enthalten sind.

**2.** Verfahren gemäß Anspruch 1, **dadurch gekennzeichnet, dass** die Färbe- oder Aufhellzusammensetzung (A) mindestens ein Polysaccharid enthält, das mit Calciumionen in wässrigem Medium ein Gel bilden kann und das bevorzugt ausgewählt ist aus Alginsäure, kappa-Carragheenan, iota-Carragheenan und Pektin, und/oder mindestens einem Salz der vorgenannten Polysaccharide, das ausgewählt ist aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist, wobei dieses mindestens eine Polysaccharid und/oder sein vorgenanntes Salz bevorzugt in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (A), enthalten ist.

**3.** Verfahren gemäß Anspruch 1 oder 2, **dadurch gekennzeichnet, dass** die Oxidationszusammensetzung (B) mindestens ein Polysaccharid enthält, das mit Calciumionen in wässrigem Medium ein Gel bilden kann und das bevorzugt ausgewählt ist aus Alginsäure, kappa-Carragheenan, iota-Carragheenan und Pektin, und/oder mindestens einem Salz der vorgenannten Polysaccharide, das ausgewählt ist aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist, wobei dieses mindestens eine Polysaccharid und/oder sein vorgenanntes Salz bevorzugt in einer Gesamtmenge von 0,1 - 5 Gew.-%, besonders bevorzugt-0,2 - 3 Gew.-%, außerordentlich bevorzugt 0,5 - 2,5 Gew.-%, jeweils bezogen auf das Gewicht der Zusammensetzung (B), enthalten ist.

**4.** Verfahren gemäß einem der Ansprüche 1 - 3, **dadurch gekennzeichnet, dass** das mindestens eine Polysaccharid, das mit Calciumionen in wässrigem Medium ein Gel bilden kann und das bevorzugt ausgewählt ist aus Alginsäure, kappa-Carragheenan, iota-Carragheenan und Pektin, und/ oder mindestens einem Salz der vorgenannten Polysaccharide, das ausgewählt ist aus den. Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist, bereitgestellt wird in Form einer Polysaccharid-Zusammensetzung (D), enthaltend

d)i. mindestens ein Polysaccharid, das mit Calciumionen in wässrigem Medium ein Gel bilden kann und bevorzugt ausgewählt ist aus Alginsäure, kappa-Carragheenan, iota-Carragheenan und Pektin, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, bevorzugt in einer Gesamtmenge von 0,1 - 5 Gew.-%, bevorzugt 0,5 - 2 Gew.-%, besonders bevorzugt 0,5 - 2,5 Gew.-%, jeweils bezogen auf das Gewicht der Zusammensetzung (D), wobei im Falle von Salzen des kappa-Carragheenans dessen Alkalimetall-Salz nur aus Lithium- und Natriumsalzen ausgewählt ist,

d)ii. 50 - 99,9, bevorzugt 60 bis 95, besonders bevorzugt 80 bis 90 Gew.-% Wasser, jeweils bezogen auf das Gewicht der Zusammensetzung (D),

d)iii. optional mindestens ein Alkalisierungsmittel, das bevorzugt ausgewählt ist aus pulverförmigen Natriumsilikaten, insbesondere pulverförmigen Natriummetasilikaten,

d)iv. optional mindestens ein Polymer, enthaltend (Meth)Acrylsäure-, (Meth)Acrylsäureester-, (Meth)Acrylamid- und/oder 2-Acrylamido-2-methylpropansulfonsäure-Monomere, bevorzugt in einer Gesamtmenge von 0,05 - 3 Gew.-%, bevorzugt 0,2 - 0,5 Gew.-%, bezogen auf das Gewicht der Zusammensetzung (D),

d)v. optional mindestens ein Öl in einer Gesamtmenge von 0,1 bis 10 Gew.-%, bevorzugt 0,3 bis 5 Gew.-%, besonders bevorzugt 1 bis 3 Gew.-%, jeweils bezogen auf das Gewicht der Polysaccharid-Zusammensetzung (D),

wobei optional die Polysaccharid-Zusammensetzung (D) durch Vermischen einer festen, bevorzugt pulverförmigen, Polysaccharid-Zusammensetzung (D'), enthaltend d)i und optional d)iii und/oder optional d)iv, mit der Komponente d)ii und optional mit der Komponente d)v *in situ* oder 0,01 bis 24 Stunden vor Durchführung des erfindungsgemäßen Farbveränderungsverfahrens hergestellt wird,

**dadurch gekennzeichnet, dass** die Polysaccharid-Zusammensetzung (D) in einem Verfahrensschritt

- nach dem Applikationsschritt e und vor dem Applizieren, bevorzugt Aufsprühen, der Gelierzusammensetzung (C) auf die mit der Mischung (M) aus (A) und (B) beaufschlagte(n) keratinische(n) Faser(n) appliziert, bevorzugt aufgesprüht, wird, oder
- vor dem Applikationsschritt e zu einer der Zusammensetzungen (A) oder (B) oder zur Mischung (M) aus (A) und (B) gegeben wird oder
- nach dem Applizieren, bevorzugt Aufsprühen, der Gelierzusammensetzung (C) auf die mit der Mischung (M) aus (A) und (B) beaufschlagte(n) keratinische(n) Faser(n) appliziert, bevorzugt aufgesprüht, wird.

**5.** Verfahren gemäß einem der Ansprüche 1 - 4, **dadurch gekennzeichnet, dass** das Gewichtsverhältnis von Färbe- oder Aufhellzusammensetzung (A) zu Oxidationszusammensetzung (B) im Bereich von 1 : 10 bis 4:1, bevorzugt im Bereich von 1 : 5 bis 3 : 1, besonders bevorzugt im Bereich von 1 : 4 bis 2 : 1 und außerordentlich bevorzugt im Bereich von 1:2 bis 1:1 liegt.

**6.** Verfahren gemäß Anspruch 4 oder 5, **dadurch gekennzeichnet, dass** das Gewichtsverhältnis von Polysaccharid-Zusammensetzung (D) zur Mischung (M) aus Färbe- oder Aufhellzusammensetzung (A) und Oxidationszusammensetzung (B) im Bereich von 1 : 20 bis 1:2, bevorzugt im Bereich von 1:10 bis 1:5 liegt.

**7.** Verfahren gemäß einem der Ansprüche 1 - 6, **dadurch gekennzeichnet, dass** das Gewichtsverhältnis von Gelierzusammensetzung (C) zur Mischung (M) aus Färbe- oder Aufhellzusammensetzung (A) und Oxidationszusammensetzung (B) im Bereich von 1 : 20 bis 1:2, bevorzugt im Bereich von 1:10 bis 1:5 liegt.

**8.** Verfahren gemäß einem der Ansprüche 4 - 7, **dadurch gekennzeichnet, dass** das Gewichtsverhältnis von Polysaccharid-Zusammensetzung (D) zur Gelierzusammensetzung (C) im Bereich von 0,2 : 1 bis 2:1, bevorzugt im Bereich von 0,5 : 1 bis 1,5 : 1 liegt und besonders bevorzugt 1:1 beträgt.

**9.** Verfahren gemäß einem der Ansprüche 1 - 8, **dadurch gekennzeichnet, dass** es mit mindestens einer zweiten Färbe- oder Aufhellzusammensetzung (A'), (A"), (A"') usw. anstelle von (A) durchgeführt wird, wobei sich die Zusammensetzungen (A) und (A'), (A"), (A"') usw. zumindest in Bezug auf die enthaltenen Oxidationsfarbstoffvorprodukte und/oder direktziehenden Haarfarbstoffe qualitativ und/oder quantitativ voneinander unterscheiden.

**10.** Verfahren gemäß einem der Ansprüche 1 - 9, **dadurch gekennzeichnet, dass** die Keratinfasersträhnen nicht mit festen flächigen Trennhilfen, insbesondere nicht mit Folien aus festen Materialien, wie Aluminium, Papier oder Styropor, versehen sind.

**11.** Kit zur Farbveränderung keratinischer Fasern, umfassend die folgenden, physikalisch voneinander getrennten Komponenten (D) und (C):

I. eine Polysaccharid-Zusammensetzung (D), enthaltend

d)i. mindestens ein Polysaccharid, das mit Calciumionen in wässrigem Medium ein Gel bilden kann, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist,
d)ii. mindestens ein Alkalisierungsmittel,

II. eine Gelierzusammensetzung (C'), enthaltend
c)i. mindestens ein Salz eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, mindestens ein Salz, ausgewählt aus Kalium-, Calcium-, Strontium-, Barium- und Aluminiumsalzen mit einer Wasserlöslichkeit bei 20°C von mindestens 500 mg/l.

**12.** Verfahren gemäß einem der Ansprüche 4 - 10 oder Kit nach Anspruch 11, **dadurch gekennzeichnet, dass** die Zusammensetzung (D) mindestens ein Polymer, enthaltend (Meth)Acrylsäure-, (Meth)Acrylsäureester-, (Meth)Acrylamid- und/oder 2-Acrylamido-2-methylpropansulfonsäure-Monomere, ausgewählt aus vernetzten Homopolymeren der Acrylsäure, vernetzten Homopolymeren der 2-Acrylamido-2-methylpropansulfonsäure, vernetzten Copolymeren bestehend aus Acrylsäure- und 2-Acrylamido-2-methylpropansulfonsäure-Einheiten, vernetzten

Copolymeren bestehend aus 2-Methyl-2[(1-oxo-2-propenyl)amino]-1-propansulfonsäure-Einheiten und Hydroxyethylacrylat-Einheiten, vernetzten Copolymeren bestehend aus Acrylamid- und 2-Acrylamido-2-methyl-propansulfonsäure-Einheiten, vernetzten Copolymeren bestehend aus Methacrylsäure-Einheiten und C1-C4-Alkylacrylat-Einheiten, vernetzten Copolymeren bestehend aus Acrylsäure-Einheiten und C1-C4-Alkylmethacrylat-Einheiten, sowie Mischungen hiervon, enthält.

**13.** Verfahren gemäß einem der Ansprüche 1 - 10 oder 12, **dadurch gekennzeichnet, dass** die Färbezusammensetzung (A) mindestens ein Oxidationsfarbstoffvorprodukt, ausgewählt aus 4,5-Diamino-1-(2-hydroxyethyl)pyrazol, 4-Amino-3-methylphenol, 4-Amino-2-Hydroxytoluen und m-Aminophenol sowie Mischungen dieser Oxidationsfarbstoffvorprodukte enthält.

**14.** Verwendung von mindestens einem Polysaccharidsalz, ausgewählt aus Calciumalginat, Kalium-kappa-Carragheenat, Calcium-kappa-Carragheenat, Calcium-iota-Carragheenat, Calciumpektinat, Aluminiumalginat, Aluminium-kappa-Carragheenat, Aluminium-iota-Carragheenat, Aluminiumpektinat, Strontiumalginat, Strontium-kappa-Carragheenat, Strontium-iota-Carragheenat, Strontiumpektinat, Bariumalginat, Barium-kappa-Carragheenat, Barium-iota- Carragheenat und Bariumpektinat, sowie Mischungen dieser Polysaccharidsalze, zur Beschichtung von Keratinfaserpartien oder Keratinfasersträhnen in einem Verfahren zur Farbveränderung keratinischer Fasern gemäß einem der Ansprüche 1 - 10, 12 oder 13.

## Claims

**1.** A method for changing the color of keratin fibers, **characterized by** the following method steps:

a. providing a coloring or lightening composition (A) containing

a)i. at least one alkalizing agent,
a)v. the coloring or lightening composition (A), if it contains more than 7 wt.% water, based on the weight thereof, having a pH in the range of from 6.0 to 11.5, measured at 20 °C, and if the composition (A) contains 0 to 7 wt.% water, based on the weight thereof, it being composed such that the 30 wt.% dispersion thereof in water has a pH in the range of from 6.0 to 12.0, in each case measured at 20 °C;

b. providing an oxidizing composition (B) containing

b)i. 1-18 wt.% hydrogen peroxide, based on the weight of the composition (B), and
b)ii. water,
b)iii. the oxidizing composition (B) having a pH in the range of from 2.5 to 6.5, in each case measured at 20 °C;

c. providing a gelling composition (C) containing

c)i. 0.1 to 3 wt.%, based on the weight of the composition (C), of at least one salt of a polyvalent metal ion, selected from calcium, strontium, barium and aluminum salts having a water solubility at 20 °C of at least 500 mg/l, and, in the case that the polysaccharide which forms a gel with calcium ions comprises kappa-carrageenan or one of the salts thereof, the gelling composition (C) containing 0.1 to 3 wt.%, based on the weight of the composition (C), of at least one salt selected from potassium, calcium, strontium, barium and aluminum salts having a water solubility at 20 °C of at least 500 mg/l,
c)ii. 50 to 99 wt.% water, based on the weight of the composition (C),
c)iii. the gelling composition (C) having a pH in the range of from 4 to 12, measured at 20 °C;

subsequently, i.e. 1 to 600 seconds later,
d. preparing a mixture (M) from (A) and (B) which has a pH in the range of from 6.0 to 11, in each case measured at 20 °C; subsequently, after 1 to 600 seconds,
e. applying at least a partial amount of (M) to at least one keratin fiber section to be colored and/or lightened; 1 to 600 seconds later
f. applying the gelling composition (C) to the keratin fiber section(s) subjected to (M);
g. repeating steps e. and f. to the desired extent,
h. leaving on the keratin fibers for a time of 0.1 to 60 minutes, then rinsing the keratin fibers with water and optionally a cleaning agent, optionally post-treatment of the fibers with a conditioning agent, and subsequent

drying,

**characterized in that** the method comprises applying at least one polysaccharide which can form a gel with calcium ions in an aqueous medium, these polysaccharides not being contained in the gelling composition (C).

2. The method according to claim 1, **characterized in that** the coloring or lightening composition (A) contains at least one polysaccharide which can form a gel with calcium ions in an aqueous medium and which is preferably selected from alginic acid, kappa-carragheenan, iota-carragheenan and pectin, and/or at least one salt of the aforementioned polysaccharides which is selected from the alkali metal, ammonium, mono-, di- and trialkylammonium, mono-, di- and trialkanolammonium and magnesium salts, where, in the case of kappa-carragheenan salts, the alkali metal salt thereof is selected only from lithium and sodium salts, this at least one polysaccharide and/or the aforementioned salt thereof being contained preferably in a total amount of 0.1 to 5 wt.%, particularly preferably 0.2 to 3 wt.%, extremely preferably 0.5 to 2.5 wt.%, in each case based on the weight of the composition (A).

3. The method according to claim 1 or 2, **characterized in that** the oxidizing composition (B) contains at least one polysaccharide which can form a gel with calcium ions in an aqueous medium and which is preferably selected from alginic acid, kappa-carragheenan, iota-carragheenan and pectin, and/or at least one salt of the aforementioned polysaccharides which is selected from the alkali metal, ammonium, mono-, di- and trialkylammonium, mono-, di- and trialkanolammonium and magnesium salts, where, in the case of kappa-carragheenan salts, the alkali metal salt thereof is selected only from lithium and sodium salts, this at least one polysaccharide and/or the aforementioned salt thereof being contained preferably in a total amount of 0.1 to 5 wt.%, particularly preferably 0.2 to 3 wt.%, extremely preferably 0.5 to 2.5 wt.%, in each case based on the weight of the composition (B).

4. The method according to one of claims 1-3, **characterized in that** the at least one polysaccharide which can form a gel with calcium ions in an aqueous medium and which is preferably selected from alginic acid, kappa-carragheenan, iota-carragheenan and pectin, and/or at least one salt of the aforementioned polysaccharides which is selected from the alkali metal, ammonium, mono-, di- and trialkylammonium, mono-, di- and trialkanolammonium and magnesium salts, where, in the case of kappa-carragheenan salts, the alkali metal salt thereof is selected only from lithium and sodium salts, is provided in the form of a polysaccharide composition (D), containing

d)i. at least one polysaccharide which can form a gel with calcium ions in an aqueous medium and which is preferably selected from alginic acid, kappa-carragheenan, iota-carragheenan and pectin, and/or at least one salt of the aforementioned polysaccharides which is selected from the alkali metal, ammonium, mono-, di- and trialkylammonium, mono-, di- and trialkanolammonium and magnesium salts, preferably in a total amount of 0.1 to 5 wt.%, preferably 0.5 to 2 wt.%, particularly preferably 0.5 to 2.5 wt.%, in each case based on the weight of the composition (D), where, in the case of kappa-carragheenan salts, the alkali metal salt thereof is selected only from lithium and sodium salts,
d)ii. 50 to 99.9, preferably 60 to 95, particularly preferably 80 to 90 wt.% water, in each case based on the weight of the composition (D),
d)iii. optionally at least one alkalizing agent, which is preferably selected from powdered sodium silicates, in particular powdered sodium metasilicates,
d)iv. optionally at least one polymer containing (meth)acrylic acid monomers, (meth)acrylic acid ester monomers, (meth)acrylamide monomers and/or 2-acrylamido-2-methylpropane sulfonic acid monomers, preferably in a total amount of 0.05 to 3 wt.%, preferably 0.2 to 0.5 wt.%, based on the weight of the composition (D),
d)v. optionally at least one oil in a total amount of 0.1 to 10 wt.%, preferably 0.3 to 5 wt.%, particularly preferably 1 to 3 wt.%, in each case based on the weight of the polysaccharide composition (D),

the polysaccharide composition (D) optionally being prepared by mixing a solid, preferably powdered, polysaccharide composition (D') containing d)i and optionally d)iii and/or optionally d)iv with component d)ii and optionally with component d)v *in situ* or 0.01 to 24 hours before the color changing method according to the invention is carried out, **characterized in that** the polysaccharide composition (D), in a method step,

- is applied, preferably sprayed, onto the keratin fiber(s), which has/have been subjected to the mixture (M) of (A) and (B), after application step e and before the application, preferably spraying, of the gelling composition (C), or
- is added to one of the compositions (A) or (B) or to the mixture (M) of (A) and (B) before application step e, or
- is applied, preferably sprayed, onto the keratin fiber(s), which has/have been subjected to the mixture (M) of (A) and (B), before the application, preferably spraying, of the gelling composition (C).

**5.** The method according to one of claims 1-4, **characterized in that** the weight ratio of coloring or lightening composition (A) to oxidizing composition (B) is in the range of from 1:10 to 4:1, preferably in the range of from 1:5 to 3:1, particularly preferably in the range of from 1:4 to 2:1 and extremely preferably in the range of from 1:2 to 1:1.

**6.** The method according to claim 4 or 5, **characterized in that** the weight ratio of polysaccharide composition (D) to the mixture (M) of coloring or lightening composition (A) and oxidizing composition (B) is in the range of from 1:20 to 1:2, preferably in the range of from 1:10 to 1:5.

**7.** The method according to one of claims 1-6, **characterized in that** the weight ratio of gelling composition (C) to the mixture (M) of coloring or lightening composition (A) and oxidizing composition (B) is in the range of from 1:20 to 1:2, preferably in the range of from 1:10 to 1:5.

**8.** The method according to one of claims 4-7, **characterized in that** the weight ratio of polysaccharide composition (D) to gelling composition (C) is in the range of from 0.2:1 to 2:1, preferably in the range of from 0.5:1 to 1.5:1 and particularly preferably is 1:1.

**9.** The method according to one of claims 1-8, **characterized in that** it is carried out with at least one second coloring or lightening composition (A'), (A"), (A"') etc. instead of (A), the compositions (A) and (A'), (A"), (A"') etc. differing qualitatively and/or quantitatively from one another, at least with regard to the oxidation dye precursors and/or substantive hair dyes contained.

**10.** The method according to one of claims 1-9, **characterized in that** the keratin fiber strands are not provided with solid, flat separating aids, in particular not with foils made of solid materials such as aluminum, paper or styrofoam.

**11.** A kit for changing the color of keratin fibers, comprising the following physically separate components (D) and (C):

I. a polysaccharide composition (D) containing

d)i. at least one polysaccharide which can form a gel with calcium ions in an aqueous medium, and/or at least one salt of the aforementioned polysaccharides which is selected from the alkali metal, ammonium, mono-, di- and trialkylammonium, mono-, di- and trialkanolammonium and magnesium salts, where, in the case of kappa-carragheenan salts, the alkali metal salt thereof is selected only from lithium and sodium salts,
d)ii. at least one alkalizing agent,

II. a gelling composition (C') containing
c)i. at least one salt of a polyvalent metal ion, selected from calcium, strontium, barium and aluminum salts having a water solubility at 20 °C of at least 500 mg/l, and, in the case that the polysaccharide which forms a gel with calcium ions comprises kappa-carrageenan or one of the salts thereof, at least one salt, selected from potassium, calcium, strontium, barium and aluminum salts having a water solubility at 20 °C of at least 500 mg/l.

**12.** The method according to one of claims 4-10 or the kit according to claim 11, **characterized in that** the composition (D) contains at least one polymer containing (meth)acrylic acid monomers, (meth)acrylic acid ester monomers, (meth)acrylamide monomers and/or 2-acrylamido-2-methylpropane sulfonic acid monomers, selected from crosslinked homopolymers of acrylic acid, crosslinked homopolymers of 2-acrylamido-2-methylpropane sulfonic acid, crosslinked copolymers consisting of acrylic acid units and 2-acrylamido-2-methylpropane sulfonic acid units, crosslinked copolymers consisting of 2-methyl-2[(1-oxo-2-propenyl)amino]-1-propane sulfonic acid units and hydroxyethyl acrylate units, crosslinked copolymers consisting of acrylamide units and 2-acrylamido-2-methyl-propane sulfonic acid units, crosslinked copolymers consisting of methacrylic acid units and C1-C4-alkyl acrylate units, crosslinked copolymers consisting of acrylic acid units and C1-C4-alkyl methacrylate units, and mixtures thereof.

**13.** The method according to one of claims 1-10 or 12, **characterized in that** the coloring composition (A) contains at least one oxidation dye precursor selected from 4,5-diamino-1-(2-hydroxyethyl)pyrazole, 4-amino-3-methylphenol, 4-amino-2-hydroxytoluene and m-aminophenol and mixtures of these oxidation dye precursors.

**14.** The use of at least one polysaccharide salt selected from calcium alginate, potassium kappa-carragheenate, calcium kappa-carragheenate, calcium iota-carragheenate, calcium pectinate, aluminum alginate, aluminum kappa-carragheenate, aluminum iota-carragheenate, aluminum pectinate, strontium alginate, strontium kappa-carragheenate, strontium iota-carragheenate, strontium pectinate, barium alginate, barium kappa-carragheenate, barium iota-carragheenate and barium pectinate, as well as mixtures of these polysaccharide salts, for coating keratin fiber sections or keratin fiber strands in a method for changing the color of keratin fibers according to one of claims 1-10, 12 or 13.

## Revendications

**1.** Procédé de changement de couleur de fibres kératiniques, **caractérisé par** les étapes de procédé suivantes :

a. fourniture d'une composition colorante ou éclaircissante (A) contenant

a)i. au moins un agent d'alcalinisation,
a)v. la composition colorante ou éclaircissante (A), si elle contient plus de 7 % en poids d'eau par rapport à son poids, présentant un pH dans la plage comprise entre 6,0 et 11,5, mesuré à 20 °C, et la composition (A), si elle contient de 0 à 7 % en poids d'eau par rapport à son poids, étant composée de telle sorte que sa dispersion de 30 % en poids dans l'eau présente un pH dans la plage comprise entre 6,0 et 12,0, respectivement mesuré à 20 °C ;

b. fourniture d'une composition d'oxydation (B) contenant

b)i 1 à 18 % en poids de peroxyde d'hydrogène par rapport au poids de la composition (B), et
b)ii de l'eau,
b)iii la composition d'oxydation (B) présentant un pH dans la plage comprise entre 2,5 et 6,5, respectivement mesuré à 20 °C ;

c. fourniture d'une composition gélifiante (C) contenant

c)i. de 0,1 à 3 % en poids, par rapport au poids de la composition (C), d'au moins un sel d'un ion métallique polyvalent choisi parmi les sels de calcium, de strontium, de baryum et d'aluminium et comportant une solubilité dans l'eau à 20 °C d'au moins 500 mg/l, et si le polysaccharide formant un gel avec des ions calcium comprend du kappa-carraghénane ou l'un de ses sels, la composition gélifiante (C) contenant de 0,1 à 3 % en poids, par rapport au poids de la composition (C), d'au moins un sel choisi parmi les sels de potassium, de calcium, de strontium, de baryum et d'aluminium et comportant une solubilité dans l'eau à 20 °C d'au moins 500 mg/l,
c)ii. de 50 à 99 % en poids d'eau par rapport au poids de la composition (C),
c)iii. la composition gélifiante (C) présentant un pH dans la plage comprise entre 4 et 12, mesuré à 20 °C ;

puis, c'est-à-dire 1 à 600 secondes plus tard,
d. préparation d'un mélange (M) de (A) et (B), présentant un pH dans la plage comprise entre 6,0 et 11, respectivement mesuré à 20 °C ; ensuite, 1 à 600 secondes plus tard,
e. application d'au moins une quantité partielle de (M) sur au moins une section de fibres kératiniques à colorer et/ou éclaircir ;
puis, 1 à 600 secondes plus tard,
f. application de la composition gélifiante (C) sur la ou les sections de fibres kératiniques exposées (M) ;
g. répétition des étapes e. et f. dans la mesure souhaitée,
h. pose, pendant une durée de 0,1 à 60 minutes, sur les fibres kératiniques, puis rinçage des fibres kératiniques avec de l'eau et éventuellement un agent nettoyant, éventuellement post-traitement des fibres au moyen d'un agent de conditionnement et séchage ultérieur,

**caractérisé en ce que** le procédé comprend l'application d'au moins un polysaccharide pouvant former un gel avec des ions calcium en milieu aqueux, ces polysaccharides n'étant pas contenus dans la composition gélifiante (C).

**2.** Procédé selon la revendication 1, **caractérisé en ce que** la composition colorante ou éclaircissante (A) contient au moins un polysaccharide pouvant former un gel avec des ions calcium en milieu aqueux et étant choisi préférablement parmi l'acide alginique, le kappa-carraghénane, l'iota-carraghénane et la pectine, et/ou au moins un sel des polysaccharides précités, choisi parmi les sels de métal alcalin, les sels d'ammonium, les sels de mono-, di- et trialkylammonium, les sels de mono-, di- et trialkanolammonium et les sels de magnésium, le sel de métal alcalin étant choisi uniquement parmi les sels de lithium et de sodium dans le cas des sels de kappa-carraghénane, ledit polysaccharide et/ou son sel précité étant contenus en une quantité totale de 0,1 à 5 % en poids, de manière particulièrement préférée de 0,2 à 3 % en poids, de manière extrêmement préférée de 0,5 à 2,5 % en poids, respectivement par rapport au poids de la composition (A).

**3.** Procédé selon la revendication 1 ou 2, **caractérisé en ce que** la composition d'oxydation (B) contient au moins un polysaccharide pouvant former un gel avec des ions calcium en milieu aqueux et étant choisi préférablement parmi l'acide alginique, le kappa-carraghénane, l'iota-carraghénane et la pectine, et/ou au moins un sel des polysaccharides précités, choisi parmi les sels de métal alcalin, les sels d'ammonium, les sels de mono-, di- et trialkylammonium, les sels de mono-, di- et trialkanolammonium et les sels de magnésium, le sel de métal alcalin étant choisi uniquement parmi les sels de lithium et de sodium dans le cas des sels de kappa-carraghénane, ledit polysaccharide et/ou son sel précité étant contenus en une quantité totale de 0,1 à 5 % en poids, de manière particulièrement préférée de 0,2 à 3 % en poids, de manière extrêmement préférée de 0,5 à 2,5 % en poids, respectivement par rapport au poids de la composition (B).

**4.** Procédé selon la revendication 1 à 3, **caractérisé en ce que** l'au moins un polysaccharide, lequel peut former un gel avec des ions calcium en milieu aqueux et est choisi préférablement parmi l'acide alginique, le kappa-carraghénane, l'iota-carraghénane et la pectine, et/ou au moins un sel des polysaccharides précités, choisi parmi les sels de métal alcalin, les sels d'ammonium, les sels de mono-, di- et trialkylammonium, les sels de mono-, di- et trialkanolammonium et les sels de magnésium, le sel de métal alcalin étant choisi uniquement parmi les sels de lithium et de sodium dans le cas des sels de kappa-carraghénane, est fourni sous la forme d'une composition de polysaccharide (D) contenant

d)i. au moins un polysaccharide pouvant former un gel avec des ions calcium en milieu aqueux et étant choisi préférablement parmi l'acide alginique, le kappa-carraghénane, l'iota-carraghénane et la pectine, et/ou au moins un sel des polysaccharides précités, choisi parmi les sels de métal alcalin, les sels d'ammonium, les sels de mono-, di- et trialkylammonium, les sels de mono-, di- et trialkanolammonium et les sels de magnésium, préférablement en une quantité totale de 0,1 à 5 % en poids, préférablement de 0,5 à 2 % en poids, de manière particulièrement préférée de 0,5 à 2,5 % en poids respectivement par rapport au poids de la composition (D), le sel de métal alcalin étant choisi uniquement parmi les sels de lithium et de sodium dans le cas des sels de kappa-carraghénane,
d)ii. 50 à 99,9, préférablement 60 à 95, de manière particulièrement préférée de 80 à 90 % en poids d'eau, respectivement par rapport au poids de la composition (D),
d)iii. en option, au moins un agent alcalinisant, préférablement choisi parmi des silicates de sodium en poudre, en particulier des métasilicates de sodium en poudre,
d)iv. en option, au moins un polymère contenant des monomères d'acide (méth)acrylique, des monomères d'ester (méth)acrylique, des monomères de (méth)acrylamide et/ou des monomères d'acide 2-acrylamido-2-méthylpropanesulfonique, préférablement en une quantité totale de 0,05 à 3 % en poids, préférablement de 0,2 à 0,5 % en poids, par rapport au poids de la composition (D),
d)v. en option, au moins une huile en une quantité totale de 0,1 à 10 % en poids, préférablement de 0,3 à 5 % en poids, de manière particulièrement préférée de 1 à 3 % en poids, respectivement par rapport au poids de la composition de polysaccharide (D),

la composition polysaccharidique (D) étant éventuellement préparée *in situ* ou 0,01 à 24 heures avant d'effectuer le procédé de changement de couleur selon l'invention en mélangeant une composition de polysaccharide (D') solide, préférablement pulvérulente, contenant d)i et éventuellement d)iii et/ou éventuellement d)iv, avec le composant d)ii et éventuellement avec le composant d)v,
**caractérisé en ce que** la composition de polysaccharide (D), en une étape de procédé,

- après l'étape d'application e et avant l'application, préférablement par pulvérisation, de la composition gélifiante (C), est appliquée, préférablement vaporisée sur la ou les fibres kératiniques exposées au mélange (M) de (A) et (B), ou
- avant l'étape d'application e, est ajoutée à l'une des compositions (A) ou (B) ou au mélange (M) de (A) et (B), ou
- après l'application, préférablement par pulvérisation, de la composition gélifiante (C), est appliquée, préférablement pulvérisée sur la ou les fibres kératiniques exposées au mélange (M) de (A) et (B).

**5.** Procédé selon l'une des revendications 1 à 4, **caractérisé en ce que** le rapport pondéral de la composition colorante ou éclaircissante (A) à la composition oxydante (B) se situe dans la plage comprise entre 1:10 et 4:1, préférablement dans la plage comprise entre 1:5 et 3:1, de manière particulièrement préférée dans la plage comprise entre 1:4 et 2:1 et de manière extrêmement préférée dans la plage comprise entre 1:2 et 1:1.

6. Procédé selon la revendication 4 ou 5, **caractérisé en ce que** le rapport pondéral de la composition de polysaccharide (D) au mélange (M) de composition colorante ou éclaircissante (A) et de composition oxydante (B) se situe dans la plage comprise entre 1:20 et 1:2, préférablement dans la plage comprise entre 1:10 et 1:5.

7. Procédé selon l'une des revendications 1 à 6, **caractérisé en ce que** le rapport pondéral de la composition gélifiante (C) au mélange (M) de composition colorante ou éclaircissante (A) et de composition oxydante (B) se situe dans la plage comprise entre 1:20 et 1:2, préférablement dans la plage comprise entre 1:10 et 1:5.

8. Procédé selon l'une des revendications 4 à 7, **caractérisé en ce que** le rapport pondéral de la composition polysaccharidique (D) à la composition gélifiante (C) se situe dans la plage entre 0,2:1 et 2:1, préférablement dans la plage comprise entre 0,5:1 à 1,5:1 et de manière particulièrement préférée est de 1:1.

9. Procédé selon l'une des revendications 1 à 8, **caractérisé en ce que** le procédé est effectué avec au moins une seconde composition colorante ou éclaircissante (A'), (A"), (A'"), etc. au lieu de (A), les compositions (A) et (A'), (A"), (A'"), etc. différant qualitativement et/ou quantitativement les unes des autres au moins en termes de précurseurs de colorant d'oxydation et/ou colorations capillaires à action directe présentes.

10. Procédé selon l'une des revendications 1 à 9, **caractérisé en ce que** les mèches de fibres kératiniques ne sont pas pourvus d'auxiliaires de séparation plats solides, en particulier de films constitués de matériaux solides tels que l'aluminium, le papier ou le polystyrène.

11. Kit de changement de couleur de fibres kératiniques, comprenant les composants physiquement séparés (D) et (C) suivants :

    I. une composition de polysaccharide (D) contenant

        d)i. au moins un polysaccharide pouvant former un gel avec des ions calcium en milieu aqueux et/ou au moins un sel des polysaccharides précités, choisi parmi les sels de métal alcalin, les sels d'ammonium, les sels de mono-, di- et trialkylammonium, les sels de mono-, di- et trialkanolammonium et les sels de magnésium, le sel de métal alcalin étant choisi uniquement parmi les sels de lithium et de sodium dans le cas des sels de kappa-carraghénane,
        d)ii. au moins un agent d'alcalinisation,

    II. une composition gélifiante (C') contenant
    c)i. au moins un sel d'un ion métallique polyvalent choisi parmi les sels de calcium, de strontium, de baryum et d'aluminium avec une solubilité dans l'eau à 20 °C d'au moins 500 mg/l, et si le polysaccharide formant un gel avec des ions calcium comprend du kappa-carraghénane ou l'un de ses sels, au moins un sel choisi parmi les sels de potassium, de calcium, de strontium, de baryum et d'aluminium et comportant une solubilité dans l'eau à 20 °C d'au moins 500 mg/l.

12. Procédé selon l'une des revendications 4 à 10

    - ou kit selon la revendication 11, **caractérisé en ce que** la composition (D) contient au moins un polymère contenant des monomères d'acide (méth)acrylique, d'ester d'acide (méth)acrylique, de (méth)acrylamide et/ou d'acide 2-acrylamido-2-méthylpropanesulfonique, choisi parmi les homopolymères réticulés d'acide acrylique, les homopolymères réticulés d'acide 2-acrylamido-2-méthylpropanesulfonique, les copolymères réticulés constitués de motifs acide acrylique et de motifs acide 2-acrylamido-2-méthylpropanesulfonique, les copolymères réticulés constitués de motifs acide 2-méthyl-2[(1-oxo-2-propenyl)amino]-1-propanesulfonique et de motifs hydroxyéthylacrylate, les copolymères réticulés constitués de motifs acrylamide et de motifs acide 2-acrylamido-2-méthylpropanesulfonique, les copolymères réticulés constitués de motifs acide méthacrylique et de motifs acrylate d'alkyle en $C_1$-$C_4$, les copolymères réticulés constitués de motifs acide acrylique et de motifs méthacrylate d'alkyle en $C_1$-$C_4$, et les mélanges de ceux-ci.

13. Procédé selon l'une des revendications 1 à 10 ou 12, **caractérisé en ce que** la composition colorante (A) contient au moins un précurseur de colorant d'oxydation choisi parmi le 4,5-diamino-1-(2-hydroxyéthyl)pyrazole, 4-amino-3-méthylphénol, 4-amino-2-hydroxytoluène et m-aminophénol et les mélanges de ces précurseurs de colorant d'oxydation.

**14.** Utilisation d'au moins un sel de polysaccharide choisi parmi l'alginate de calcium, le kappa-carraghénane de potassium, le kappa-carraghénane de calcium, le iota-carraghénane de calcium, le pectinate de calcium, l'alginate d'aluminium, le kappa-carraghénane d'aluminium, le iota-carraghénane d'aluminium, le pectinate d'aluminium, l'alginate de strontium, le kappa-carraghénane de strontium, le iota-carraghénane de strontium, le pectinate de strontium, l'alginate de baryum, le kappa-carraghénane de baryum, le iota-carraghénate de baryum et le pectinate de baryum, et des mélanges de ces sels de polysaccharide, pour le revêtement de sections de fibres kératiniques ou de mèches de fibres kératiniques dans un procédé de changement de couleur de fibres kératiniques selon l'une des revendications 1 à 10, 12 ou 13.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2007087978 A
- EP 2574331 A2
- WO 2014164213 A1